(19) Europäisches Patentamt / European Patent Office / Office européen des brevets

(11) **EP 4 227 665 A1**

(12) **EUROPEAN PATENT APPLICATION**
published in accordance with Art. 153(4) EPC

(43) Date of publication:
**16.08.2023 Bulletin 2023/33**

(21) Application number: **21879843.7**

(22) Date of filing: **22.09.2021**

(51) International Patent Classification (IPC):
**G01N 15/14** (2006.01)    **G01N 21/64** (2006.01)

(52) Cooperative Patent Classification (CPC):
**G01N 15/14; G01N 21/64**

(86) International application number:
**PCT/JP2021/034791**

(87) International publication number:
**WO 2022/080102 (21.04.2022 Gazette 2022/16)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **12.10.2020  JP 2020171897**

(71) Applicant: **Sony Group Corporation**
**Tokyo 108-0075 (JP)**

(72) Inventor: **OTSUKA Fumitaka**
**Tokyo 108-0075 (JP)**

(74) Representative: **D Young & Co LLP**
**120 Holborn**
**London EC1N 2DY (GB)**

(54) **SERVER SYSTEM, INFORMATION PROCESSING SYSTEM, DATA ACQUISITION CLIENT TERMINAL, DATA ANALYSIS CLIENT TERMINAL, AND INFORMATION PROCESSING METHOD**

(57)    A main object of the present disclosure is to provide an information processing system that can cope with an increase in an information processing amount in an analysis of biosamples.

The present disclosure provides a server system including an automatic analysis processing section that generates to-be-output data by an analysis process on optical intensity data or fluorescence label intensity data acquired by irradiation of a biosample with light, an analysis result data storage section that stores the to-be-output data generated on the basis of the optical intensity data or the fluorescence label intensity data, and an interactive analysis processing section that analyzes the fluorescence label intensity data on the basis of an analysis command for the to-be-output data output to an output apparatus and outputs analysis result data. In addition, the present disclosure also provides an information processing system including the server system.

FIG. 5

EP 4 227 665 A1

**Description**

[Technical Field]

The present disclosure relates to a server system, an information processing system, a data acquisition client terminal, a data analysis client terminal, and an information processing method and more particularly, relates to a server system that executes processes on optical intensity data acquired by irradiation of a biosample with light, an information processing system including the server system, a data acquisition client terminal and a data analysis client terminal included in the information processing system, and an information processing method related to the process.

[Background Art]

For example, there has been performed a procedure in which a particle population of cells, microbes, liposomes, and the like is labeled by a fluorochrome, respective particles in the particle population are irradiated with a laser beam, and the intensity and/or pattern of fluorescence generated from the excited fluorochrome are/is measured to thereby measure the characteristics of the particles. Representative examples of particle analyzing apparatuses that perform the measurement include flow cytometers.

As a technology related to a process on data, particularly, optical intensity data, acquired by a flow cytometer, for example, the following PTL 1 discloses a computer program product for processing scientific data according to a model independent of a certain particular dataset. The computer program product includes a data discovery node data structure that is present on a non-transitory computer-readable storage medium and multiple processor-executable commands that are present on a non-transitory computer-readable storage medium, and the data discovery node data structure includes particular specifications.

In addition, the following PTL 2 discloses a sample analyzing system that uses flow cytometry. The sample analyzing system includes a measurement data acquiring section that performs measurement of particles included in a measurement sample prepared by adding a reagent to a specimen to thereby acquire measurement data of the particles, an output mode information acquiring section that acquires output mode information representing an output mode of the measurement data, and an output section that outputs the measurement data in the output mode according to the output mode information.

[Citation List]

[Patent Literature]

[PTL 1]
JP 2018-527674T
[PTL 2]
JP 2020-051838A

[Summary]

[Technical Problem]

In flow cytometry, there is a tendency for the number of fluorochromes used in one-time measurement to increase, and, along with this, there is also a tendency for the information processing amount in the data analysis step to increase. Accordingly, execution of the data analysis step in those information processing apparatuses require higher specifications of those information processing apparatuses, but it is often not realistic that the information processing apparatuses have such specifications, and this is not desirable for users as well.

In addition, examples of analysis systems for data obtained by flow cytometry include analysis systems adopting a client-server method. However, the analysis systems adopting the client-server method require high operating costs in some cases. A cause for this can be that, for example, a server is active at any time even in a state where there are no users using the server, or an analysis process by the server can be executed at any time.

In addition, the analysis systems adopting the client-server method also have a problem related to processing speeds. For example, along with an increase in the number of users who execute analyses simultaneously, computation resources are depleted, and the processes can be delayed. Moreover, for example, it is difficult to realize, at high speeds, processes that require a large amount of computation resources at once, such as dimensional compression processes or clustering processes, in some cases.

In view of this, a main object of the present disclosure is to provide a technique for solving at least one of these problems. Objects of the present disclosure are not limited to this, and, for example, may be to solve any one or more problems described below in the present specification.

[Solution to Problem]

The present disclosure provides a server system including an automatic analysis processing section that generates to-be-output data by an analysis process on optical intensity data or fluorescence label intensity data acquired by irradiation of a biosample with light, an analysis result data storage section that stores the to-be-output data generated on the basis of the optical intensity data or the fluorescence label intensity data, and an interactive analysis processing section that analyzes the fluorescence label intensity data on the basis of an analysis command for the to-be-output data output to an output apparatus and outputs analysis result data.

The automatic analysis processing section may calculate fluorescence label intensity data from the optical intensity data.

A process by the automatic analysis processing section and a process by the interactive analysis processing section may be executed on mutually different computation resources.

The server system may reserve a computation resource for a process by the automatic analysis processing section and/or a process by the interactive analysis processing section in response to reception of an analysis start command.

The server system can further include a database on which analysis settings data to be used in a process by the automatic analysis processing section and/or a process by the interactive analysis processing section is stored.

The server system can further include an optical data storage section that stores the optical intensity data and/or the fluorescence label intensity data.

The optical data storage section may include two or more types of storages with different access speeds, and the server system can execute a process of migrating the optical intensity data and/or the fluorescence label intensity data stored on a storage with a higher access speed to a storage with a lower access speed, in a case where a predetermined condition is satisfied.

The to-be-output data can include at least one of a two-dimensional plot image, a spectral plot image, a one-dimensional histogram image, a two-dimensional contour plot image, a dimensional compressed image, and a clustering result display view.

The present disclosure provides an information processing system including a data acquisition client terminal that acquires optical intensity data acquired by irradiation of a biosample with light or acquires fluorescence label intensity data by a calculation process on the optical intensity data and a server system including an optical data storage section that stores the optical intensity data or the fluorescence label intensity data transmitted from the data acquisition client terminal, an automatic analysis processing section that calculates fluorescence label intensity data from the optical intensity data, an analysis result data storage section that stores the to-be-output data generated on the basis of the optical intensity data or the fluorescence label intensity data, and an interactive analysis processing section that analyzes the fluorescence label intensity data on the basis of an analysis command for the to-be-output data output to an output apparatus and outputs analysis result data.

In response to acquisition of the optical intensity data or the fluorescence label intensity data, the data acquisition client terminal can transmit the optical intensity data or the fluorescence label intensity data to the server system.

In response to acquisition of the optical intensity data or the fluorescence label intensity data, the data acquisition client terminal can perform a predetermined process on the optical intensity data or the fluorescence label intensity data, and then transmit the processed optical intensity data or the processed fluorescence label intensity data to the server system.

The server system may retain in advance analysis settings data to be used in a process by the automatic analysis processing section, and
the automatic analysis processing section may calculate the fluorescence label intensity data from the optical intensity data by using the analysis settings data.

In response to storage of the optical intensity data on the optical data storage section, the automatic analysis processing section can execute a process of calculating fluorescence label intensity data from the optical intensity data.

The information processing system may further include a data analysis client terminal including the output apparatus.

The data analysis client terminal can transmit, to the server system, the analysis command for the to-be-output data output to the output apparatus.

The data analysis client terminal can cause the output apparatus to output a window on which the to-be-output data is displayed, and accept input of the analysis command on the window.

Multiple data analysis client terminals may be able to share any one or more of optical intensity data, fluorescence intensity data, and analysis settings data in the server system.

Multiple data acquisition client terminals may be able to share analysis settings data in the server system.

In addition, the present disclosure provides a data acquisition client terminal including a data acquiring section that acquires optical intensity data acquired by irradiation of a biosample with light and a transmitting section that transmits the optical intensity data to a server system in response to acquisition of the optical intensity data. In the server system, fluorescence label intensity data is calculated from the optical intensity data.

In addition, the present disclosure provides a data analysis client terminal including a communication section that receives, from a server system, to-be-output data created by the server system on the basis of fluorescence label intensity data and a processing section that performs a process of causing an output apparatus to output the to-be-output data.

The data analysis client terminal may cause the output apparatus to output a window on which the to-be-output data is displayed, and accept input of an analysis command for the to-be-output data on the window.

In addition, the present disclosure provides an information processing method including an automatic analysis process step of performing an analysis process on optical intensity data acquired by irradiation of a biosample with light or fluorescence label intensity data calculated from the optical intensity data and generating to-be-output data, an analysis result data storage step of storing the to-be-output data generated on the basis of the optical intensity data or the fluorescence label intensity data, and an interactive analysis process step of analyzing the fluorescence label intensity data on the basis of an analysis command for the to-be-output data output to an output apparatus and outputting analysis result data.

[Brief Description of Drawings]

[FIG. 1]

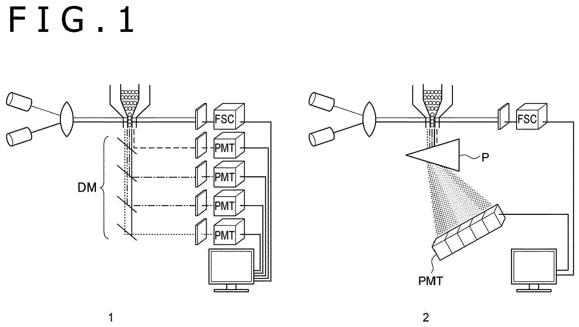

FIG. 1 is a schematic diagram of configurations of flow cytometers.
[FIG. 2]

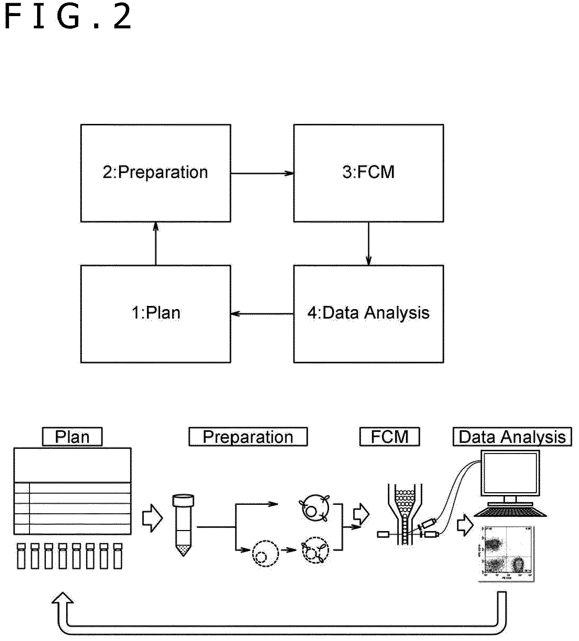

FIG. 2 is a figure depicting an experiment flow example in a case where the present technology is applied to flow cytometry.
[FIG. 3]

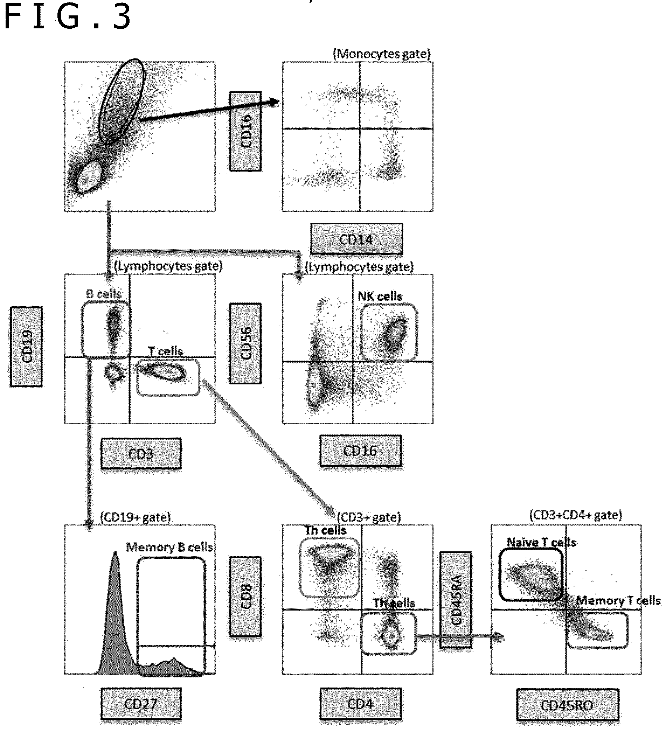

FIG. 3 is a figure depicting an example of gate setting.
[FIG. 4]

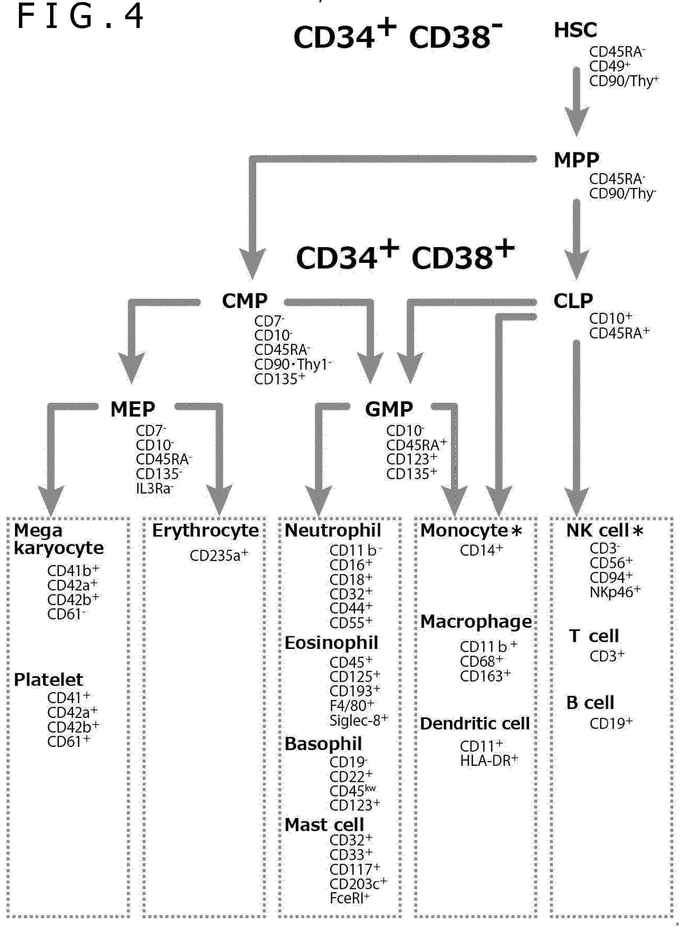

FIG. 4 is a figure for explaining a surface marker.
[FIG. 5]

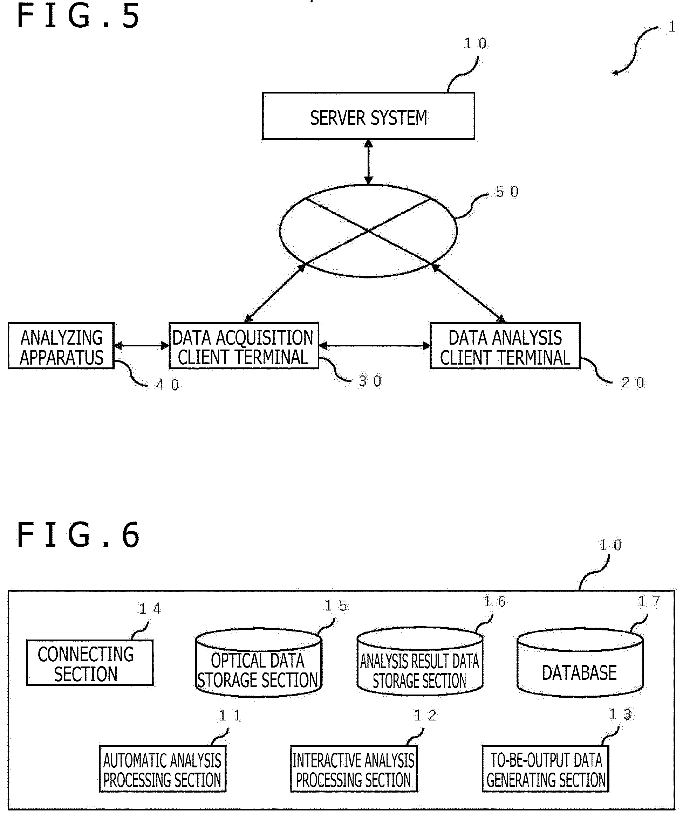

FIG. 5 is a figure depicting a configuration example of an information processing system.
[FIG. 6]
FIG. 6 is a figure depicting an example of a functional configuration of a server system.
[FIG. 7]

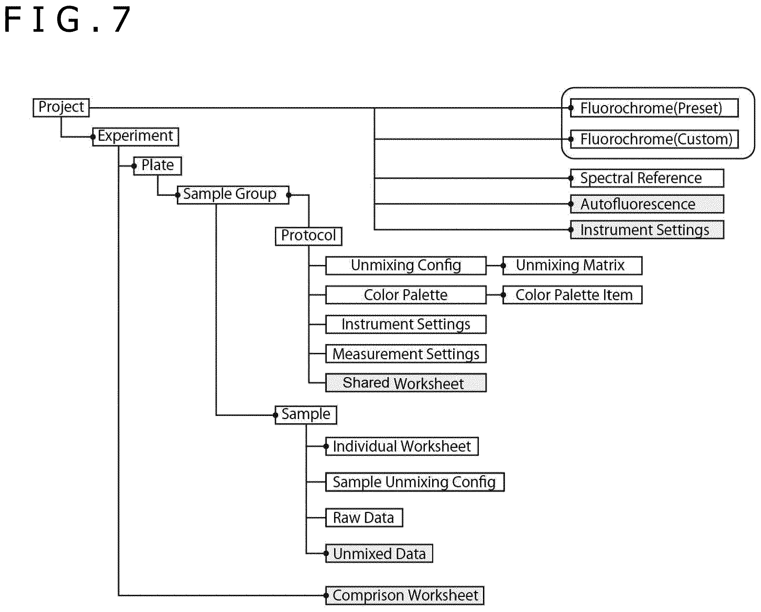

FIG. 7 is a figure depicting a configuration example of metadata.
[FIG. 8]

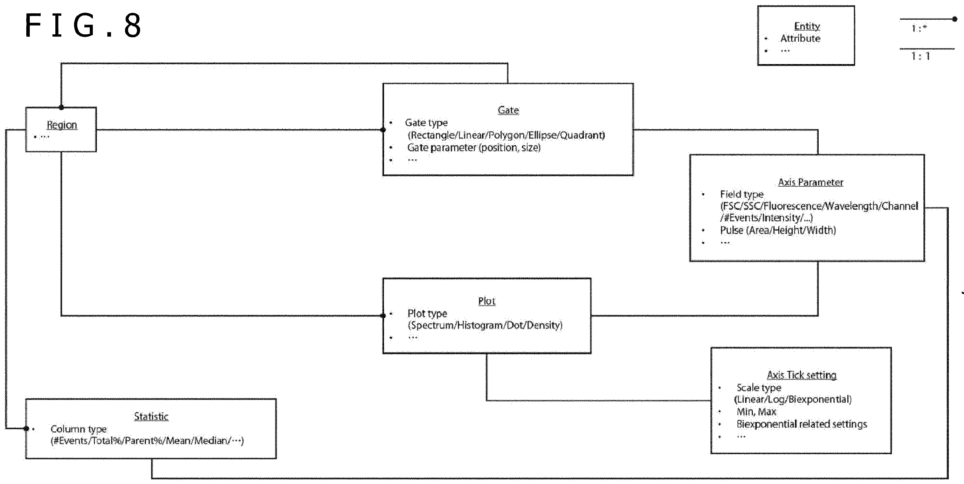

FIG. 8 is a figure depicting a configuration example of metadata.
[FIG. 9]

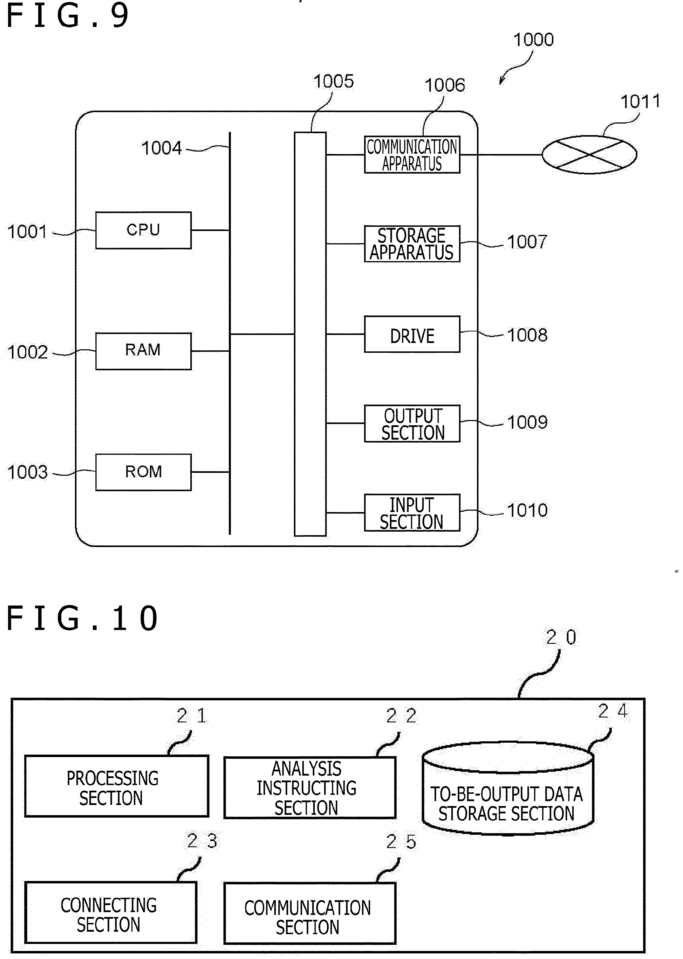

FIG. 9 is a figure depicting a hardware configuration example of a server apparatus included in the server system.
[FIG. 10]
FIG. 10 is a figure depicting an example of a functional configuration of a data analysis client terminal.
[FIG. 11]

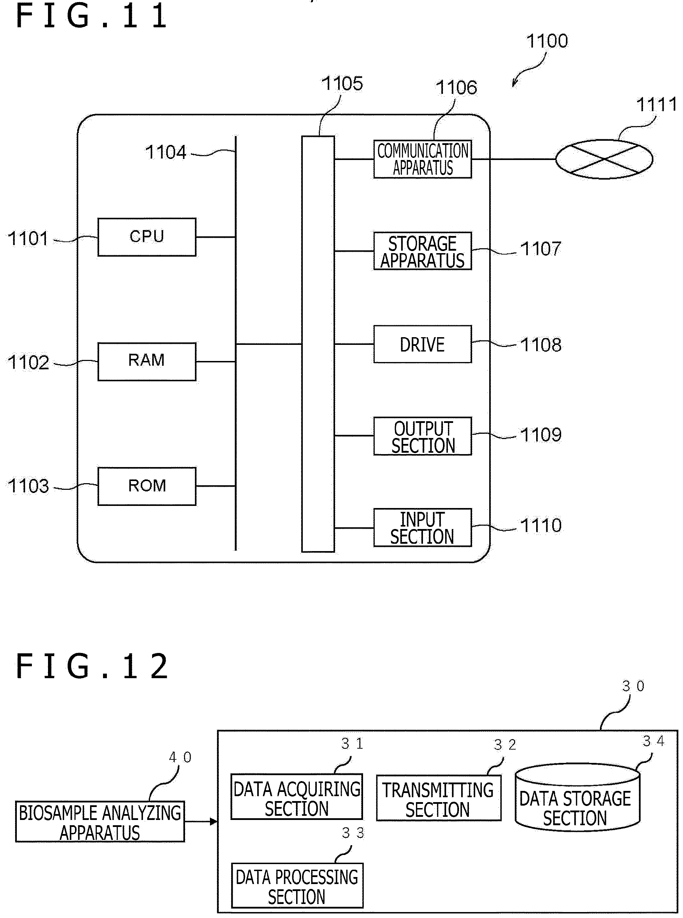

FIG. 11 is a figure depicting a hardware configuration example of the data analysis client terminal.
[FIG. 12]
FIG. 12 is a figure depicting an example of a functional configuration of a data acquisition client terminal.
[FIG. 13]

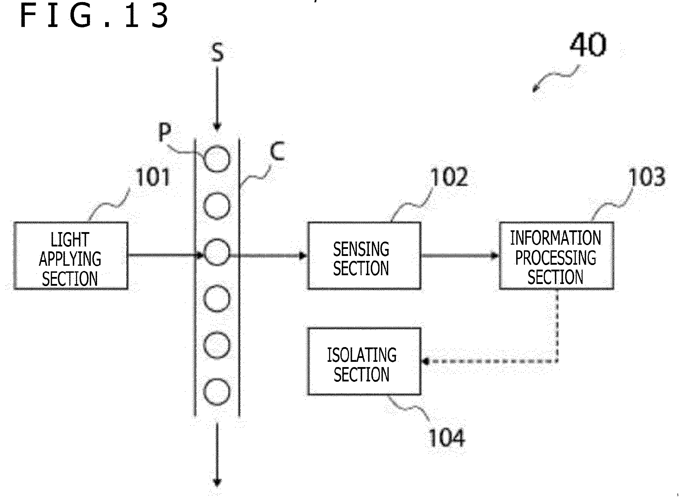

FIG. 13 is a figure depicting a configuration example of a biosample analyzing apparatus.
[FIG. 14]

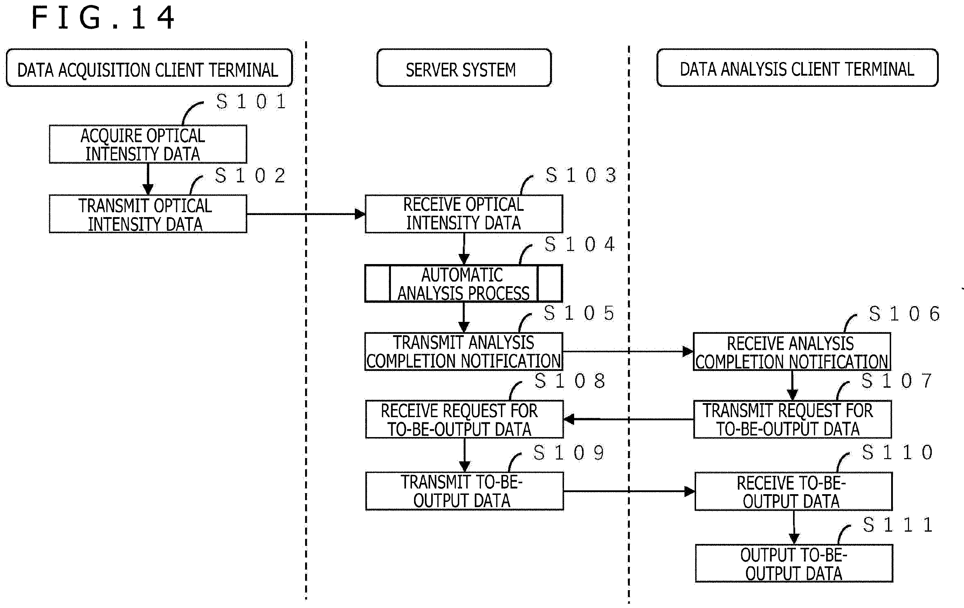

FIG. 14 is a figure depicting an example of a flow of an automatic analysis process by the information processing system.
[FIG. 15]

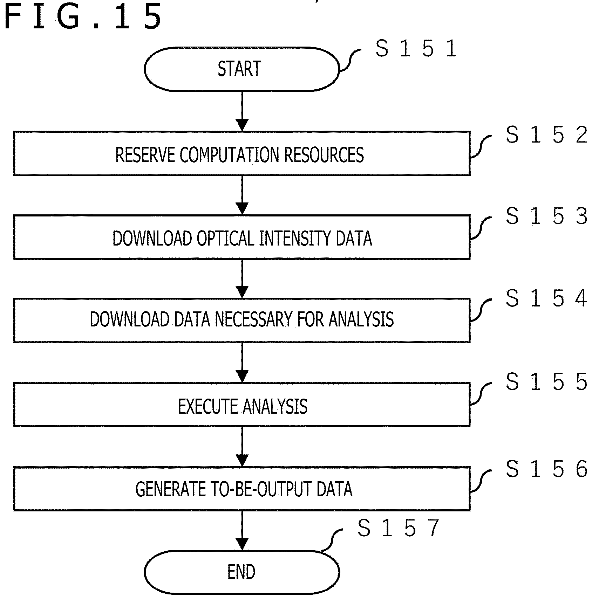

FIG. 15 is a figure depicting an example of the flow of the automatic analysis process by the information processing system.

[FIG. 16]

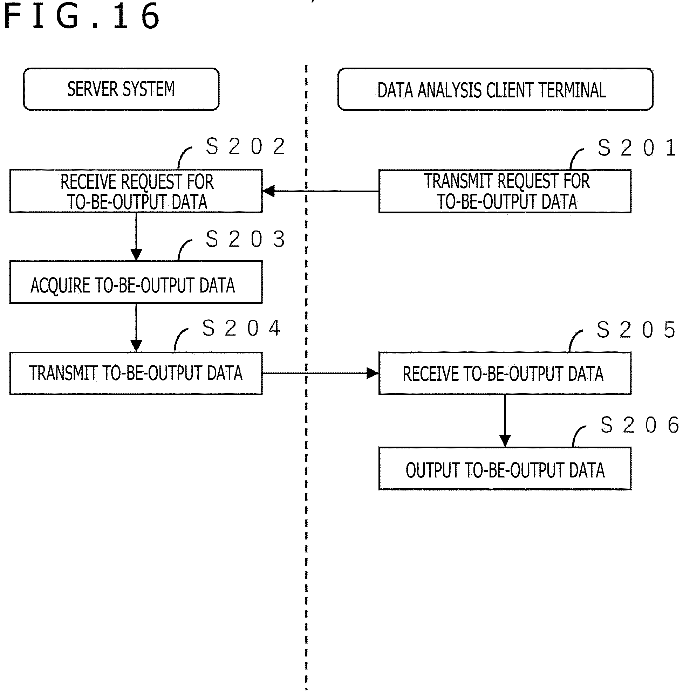

FIG. 16 is a figure depicting an example of a flow of a to-be-output data acquisition process by the information processing system.

[FIG. 17]

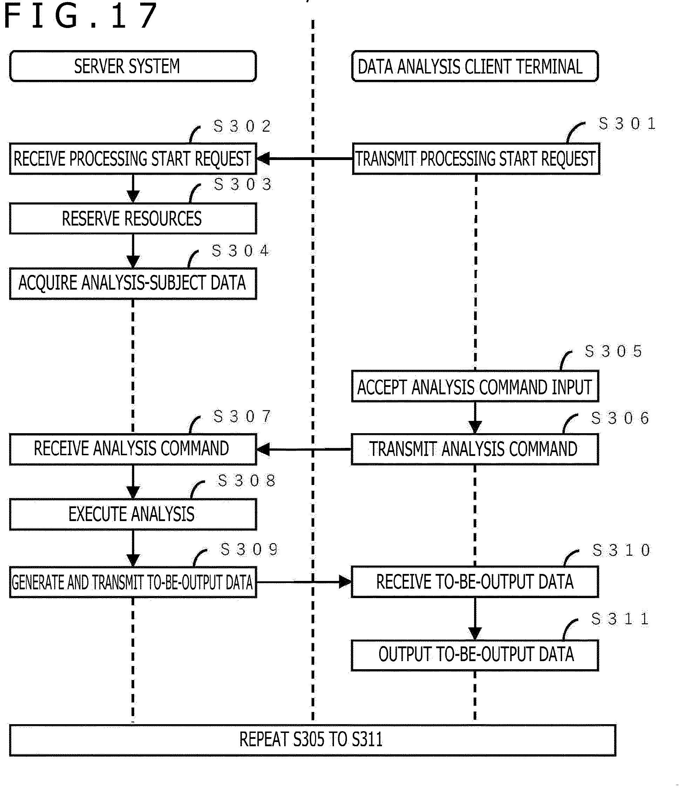

FIG. 17 is a figure depicting an example of a flow of an interactive analysis process by the information processing system.

[FIG. 18]

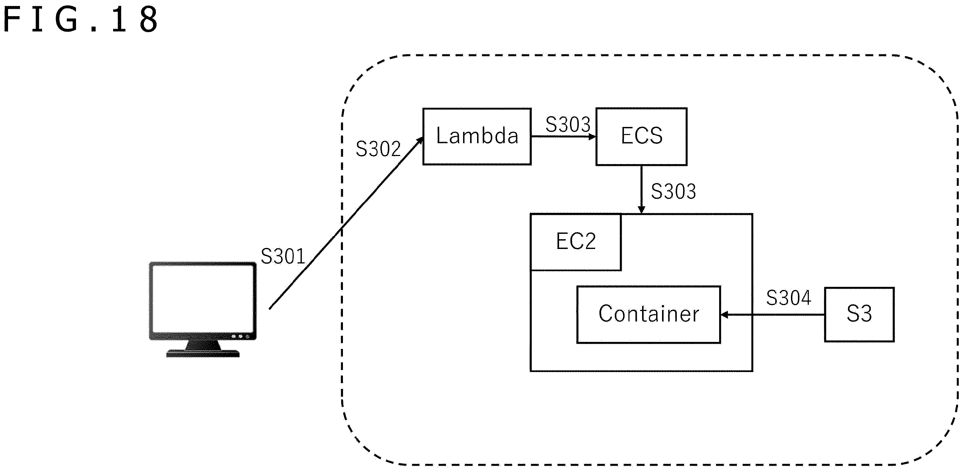

FIG. 18 is a schematic diagram for explaining an interactive analysis process flow.

[FIG. 19]

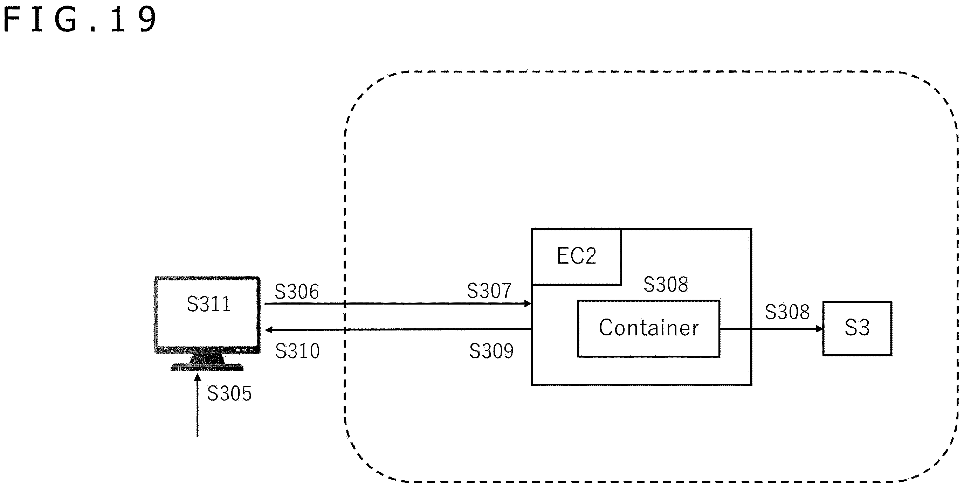

FIG. 19 is a schematic diagram for explaining the interactive analysis process flow.

[FIG. 20]

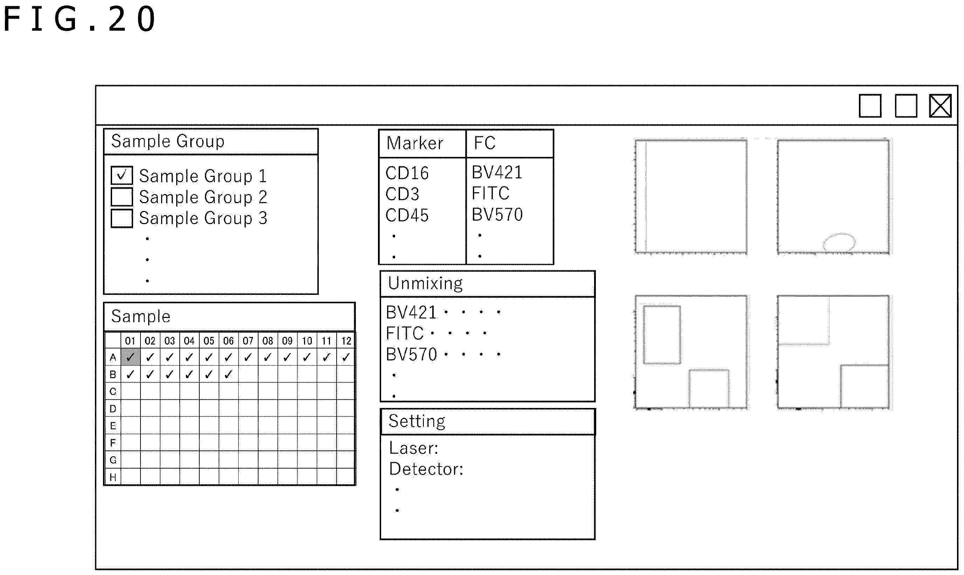

FIG. 20 is a figure depicting an example of a window that the data analysis client terminal causes an output apparatus to output.

[FIG. 21]

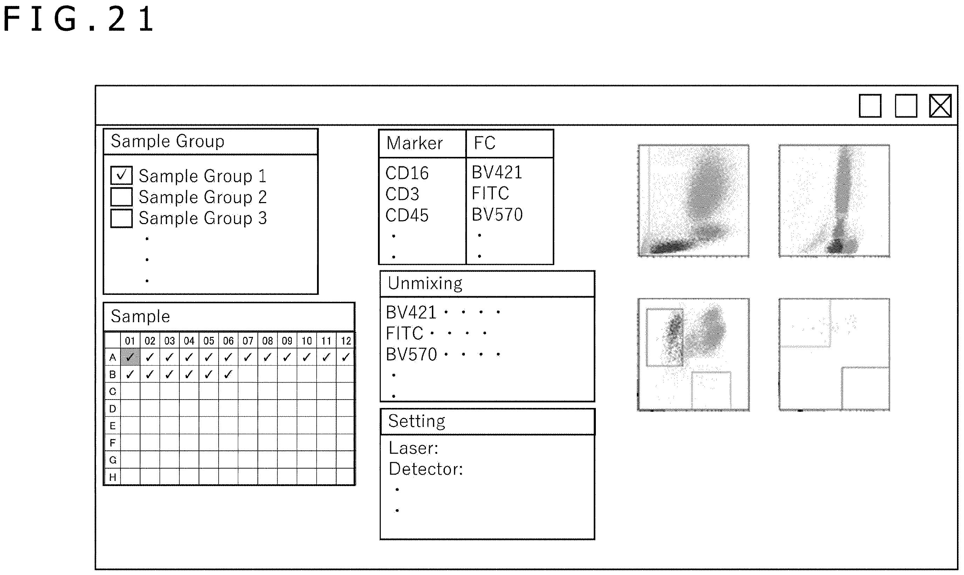

FIG. 21 is a figure depicting an example of the window that the data analysis client terminal causes the output apparatus to output.

[FIG. 22]

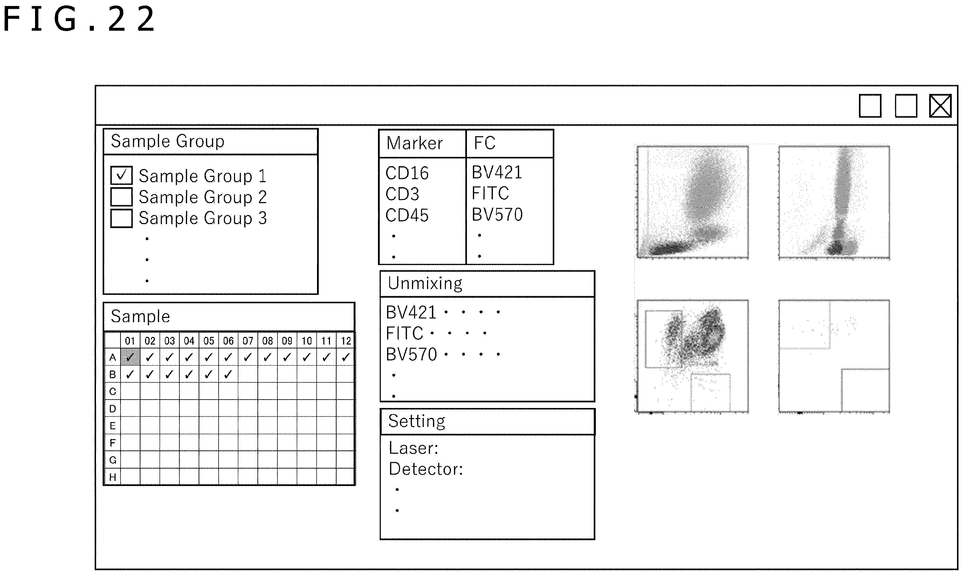

FIG. 22 is a figure depicting an example of the window that the data analysis client terminal causes the output apparatus to output.

[FIG. 23]

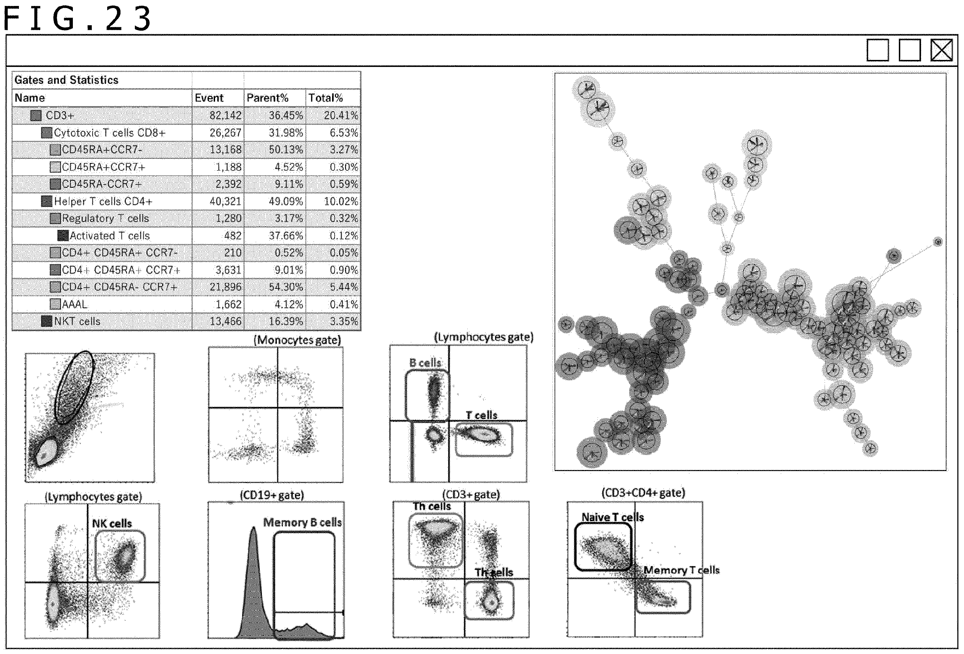

FIG. 23 is a figure depicting an example of the window that the data analysis client terminal causes the output apparatus to output.

[FIG. 24]

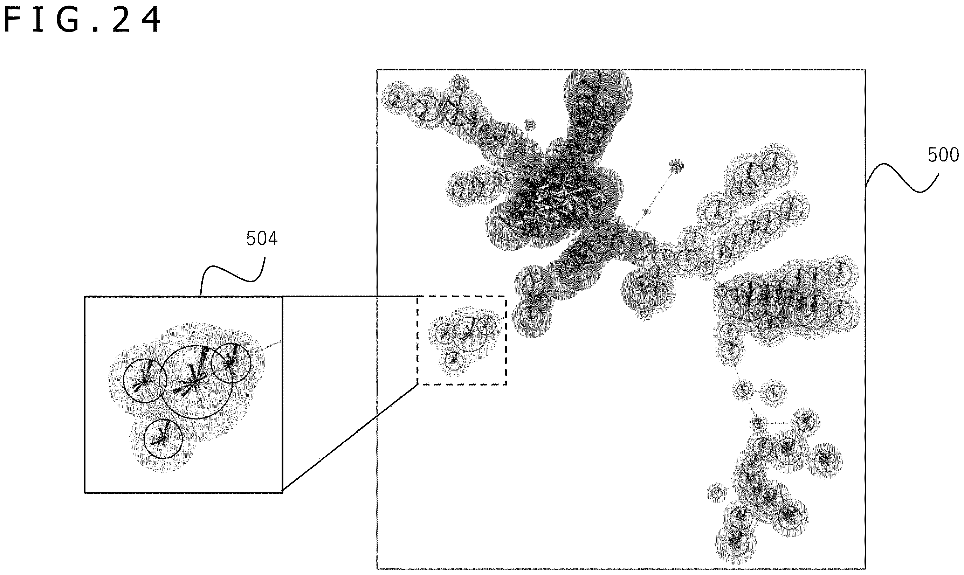

FIG. 24 is a figure depicting an example of a clustering result display view.

[FIG. 25]

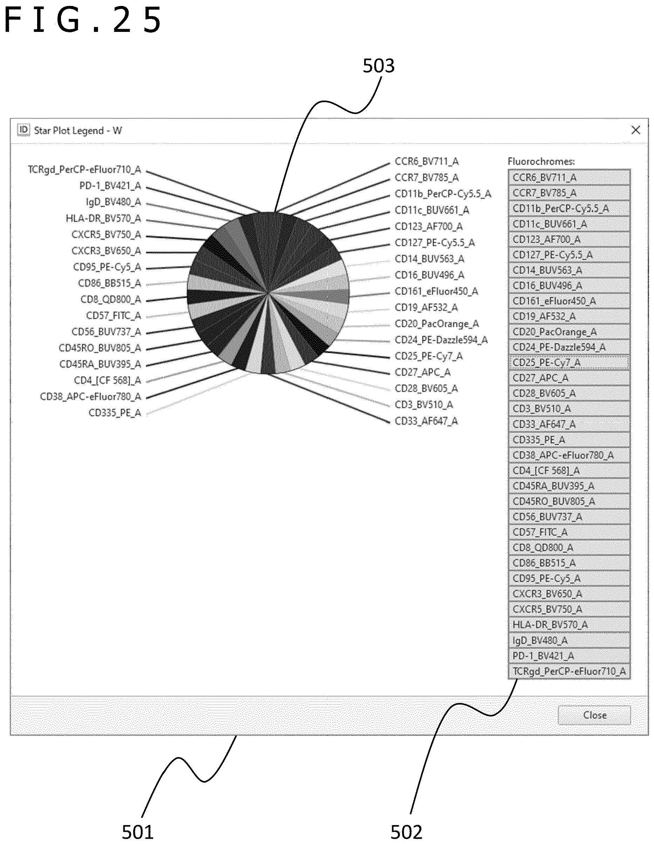

FIG. 25 is a figure depicting an example of a marker-selection window.

[FIG. 26]

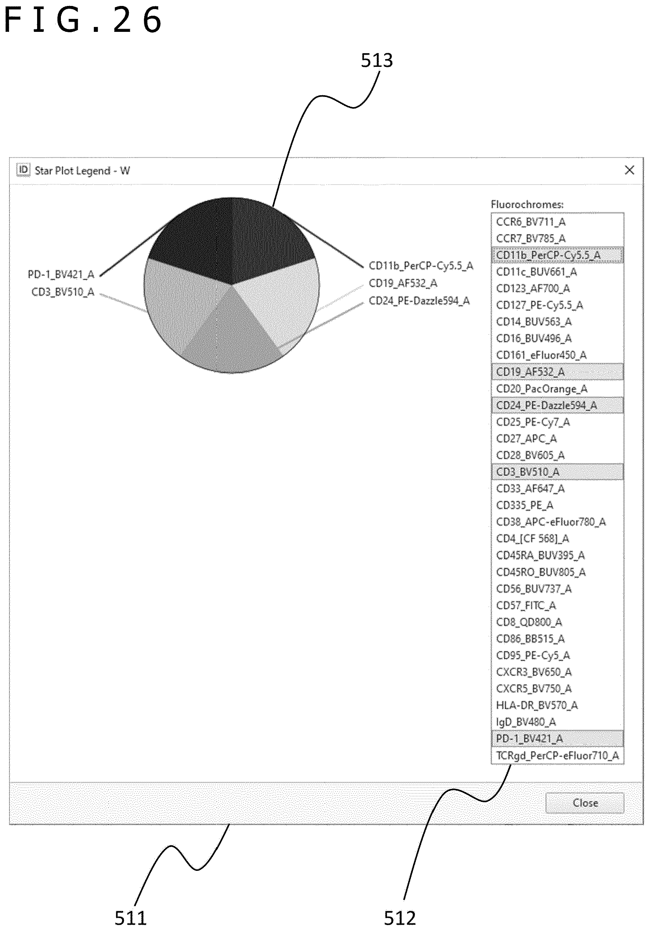

FIG. 26 is a figure depicting an example of a marker-selection window.

[FIG. 27]

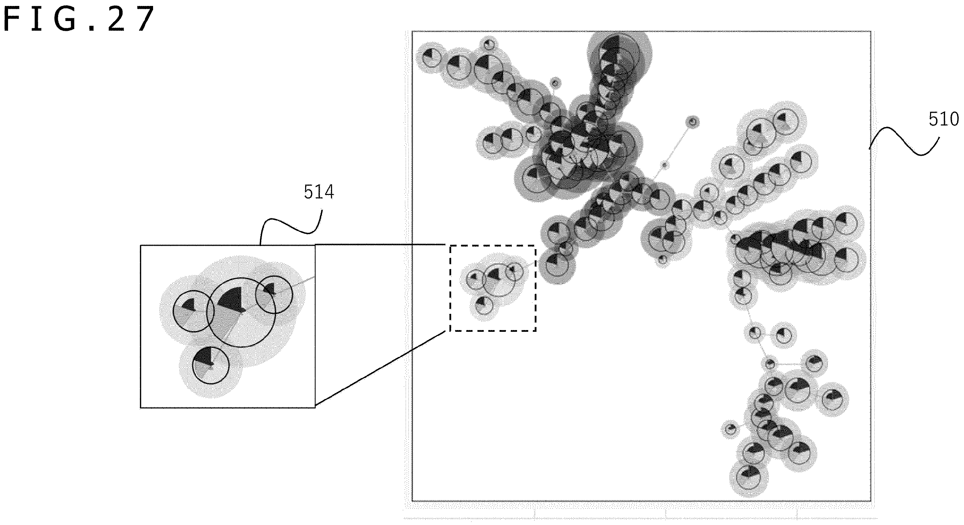

FIG. 27 is a figure depicting an example of a clustering result display view.

[FIG. 28]

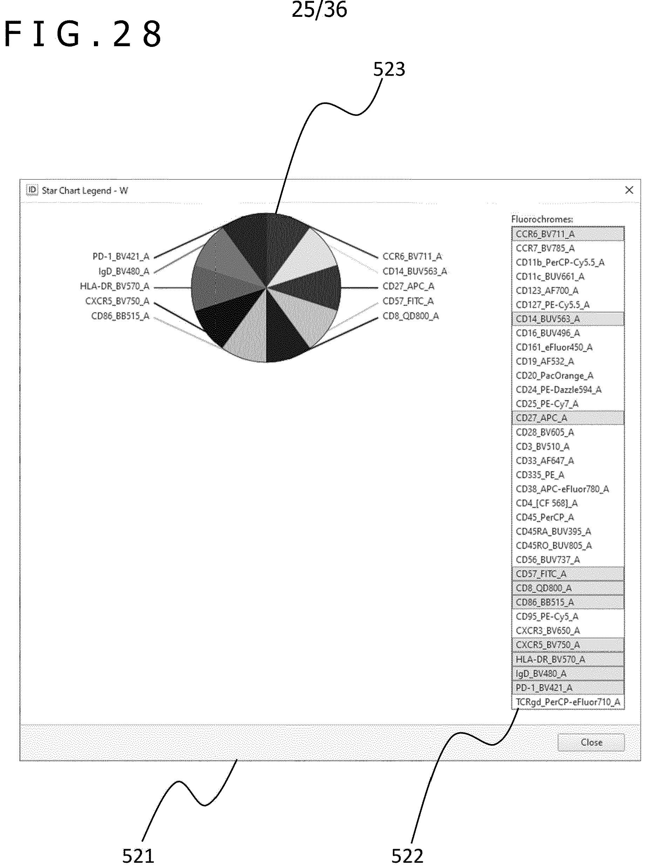

FIG. 28 is a figure depicting an example of a marker-selection window.

[FIG. 29]

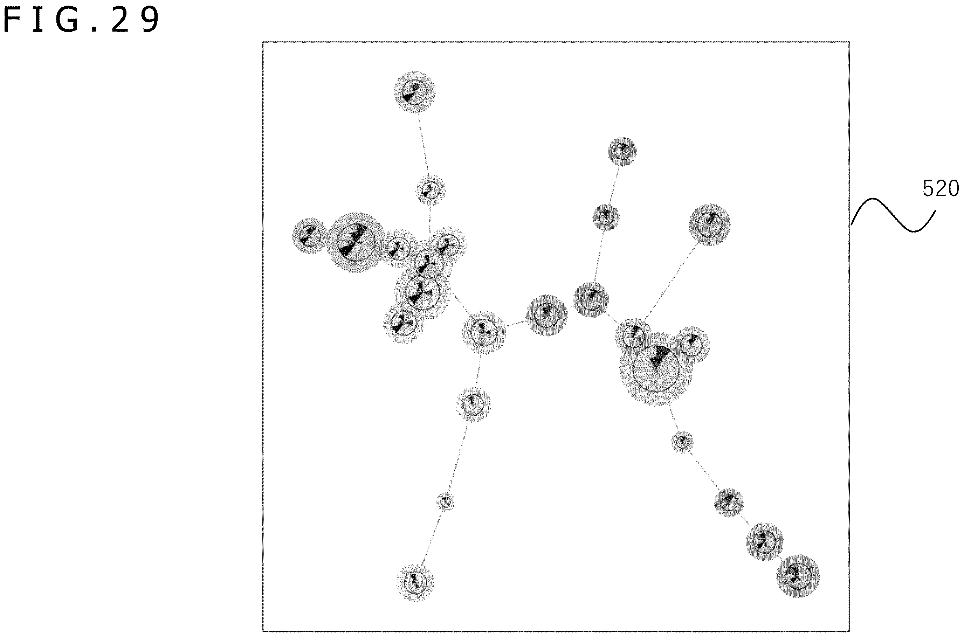

FIG. 29 is a figure depicting an example of a clustering result display view.

[FIG. 30]

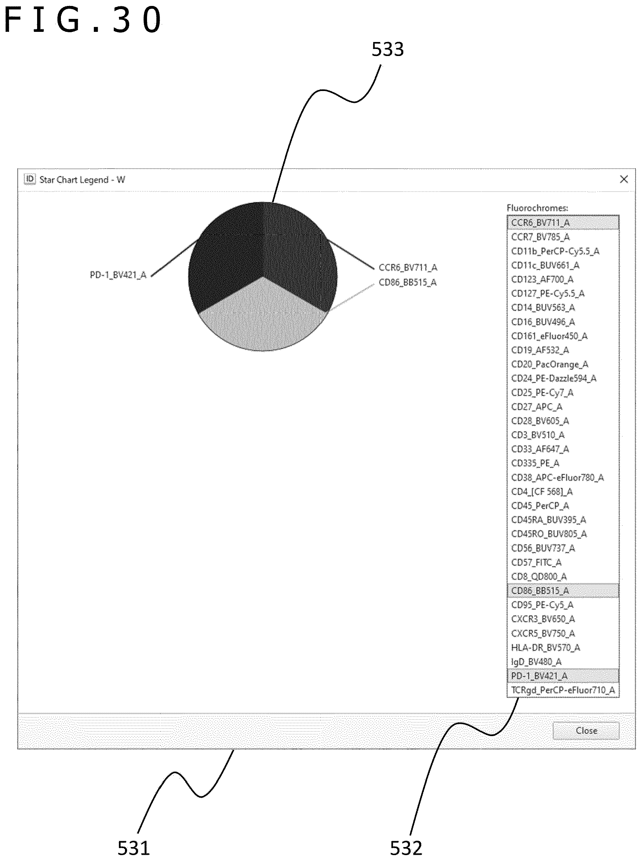

FIG. 30 is a figure depicting an example of a marker-selection window.

[FIG. 31]

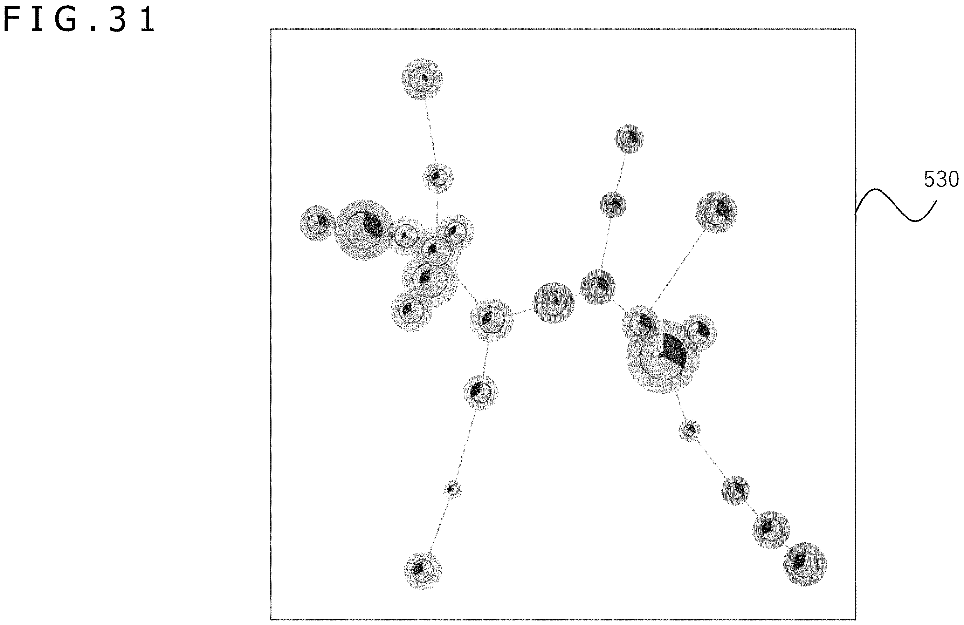

FIG. 31 is a figure depicting an example of a clustering result display view.

[FIG. 32]

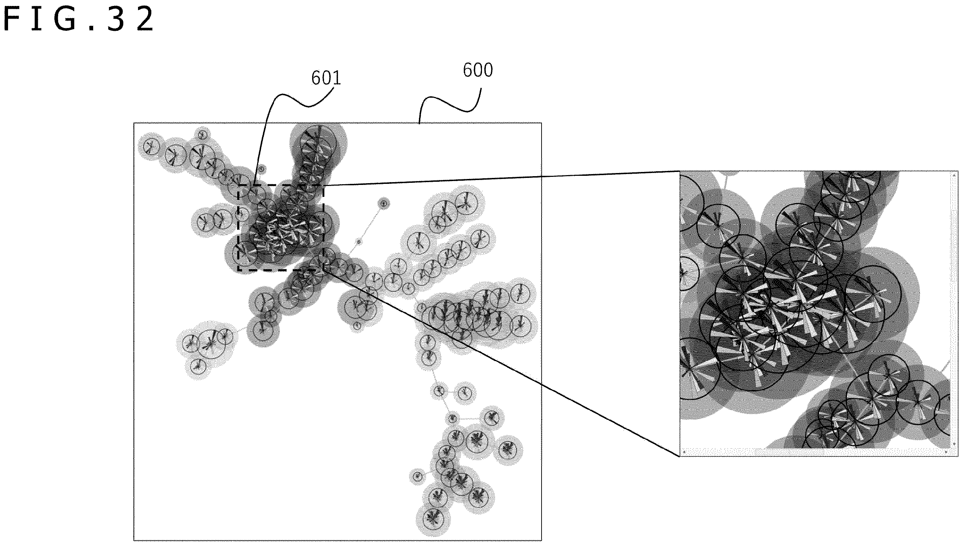

FIG. 32 is a figure depicting an example of a clustering result display view image.

[FIG. 33]

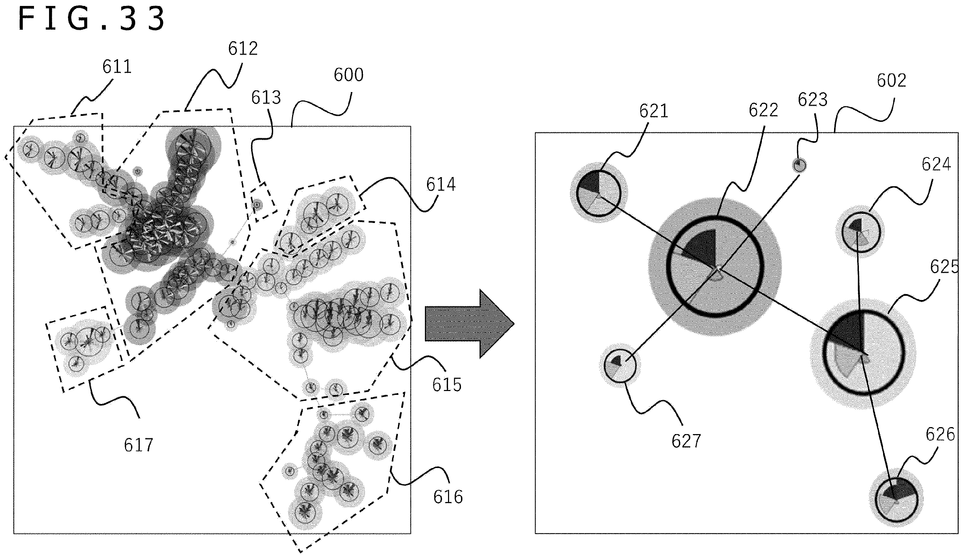

FIG. 33 is a figure depicting an example of a metacluster chart.

[FIG. 34]

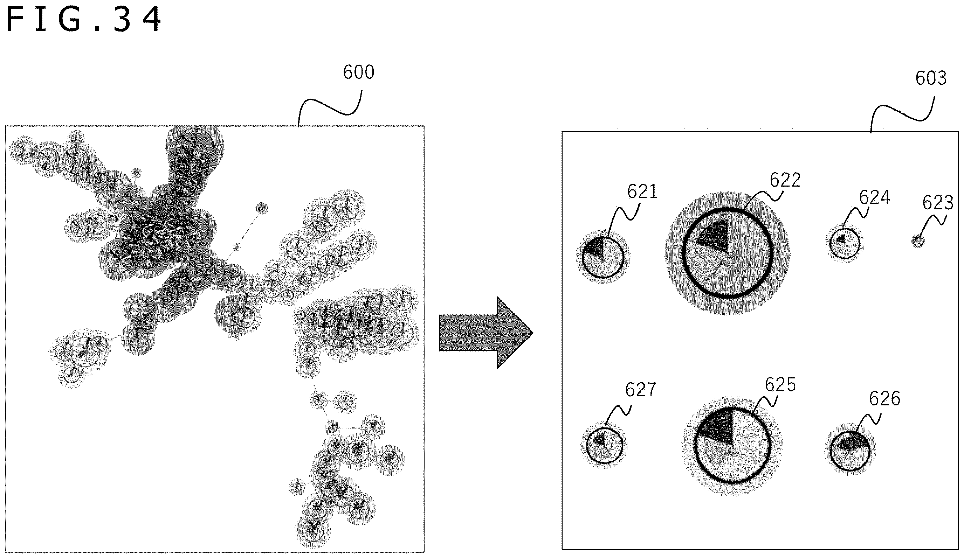

FIG. 34 is a figure depicting an example of a metacluster chart.

[FIG. 35]

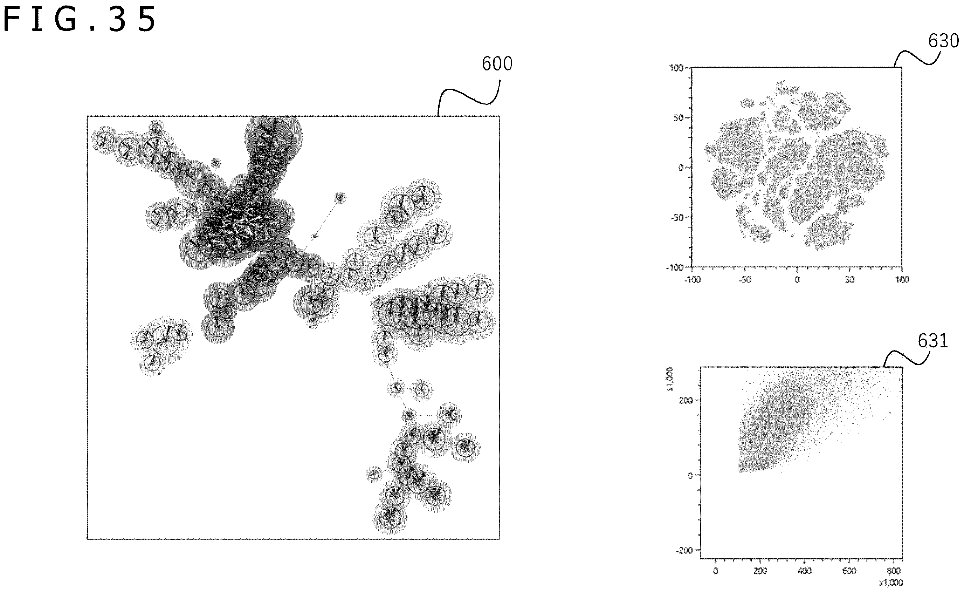

FIG. 35 is a figure depicting an example of the clustering result display view and an example of a two-dimensional plot corresponding to it.

[FIG. 36]

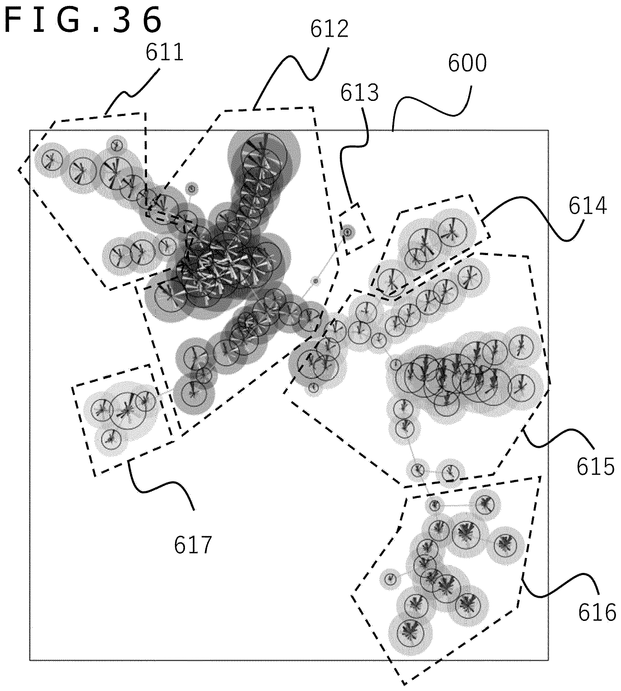

FIG. 36 is a figure for explaining metaclusters in the clustering result display view.

[FIG. 37]

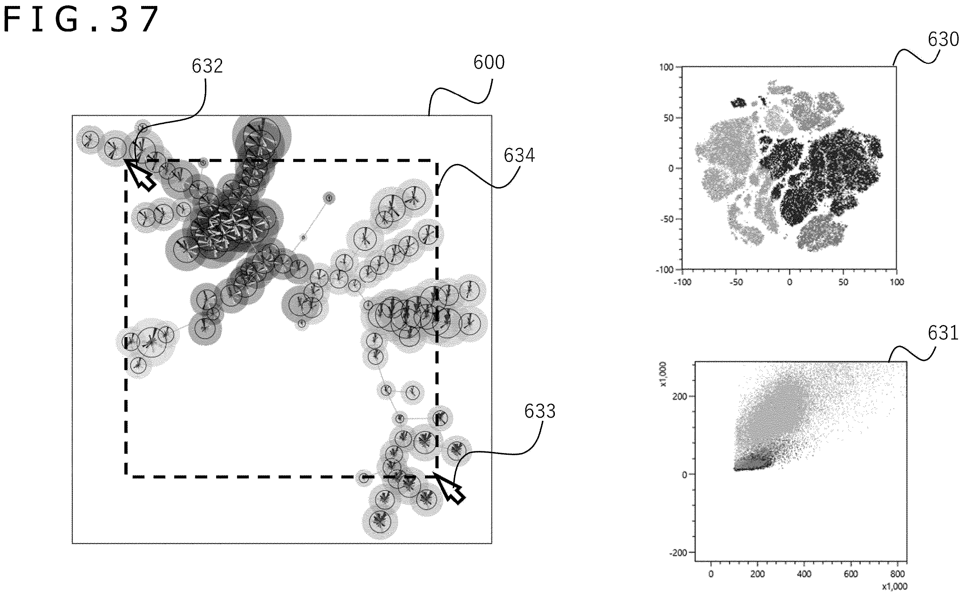

FIG. 37 is a figure depicting an example of an operation by a user on the clustering result display view and an example of a two-dimensional plot color-coded by the operation.

[FIG. 38]

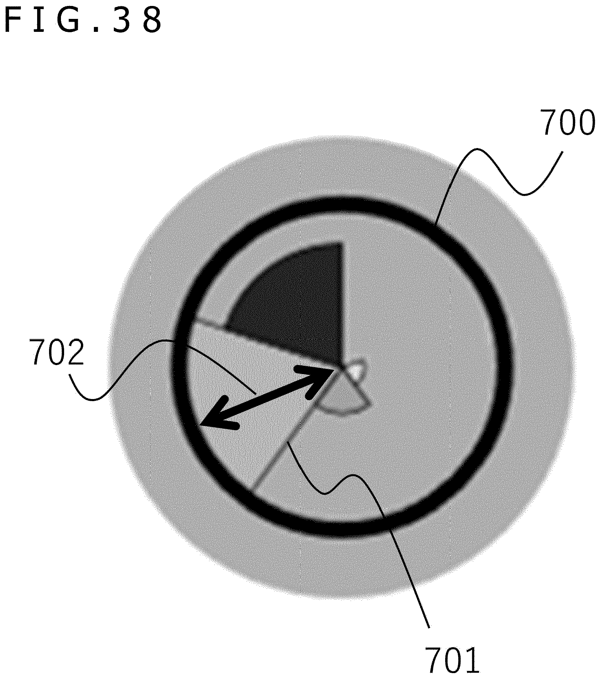

FIG. 38 is a figure for explaining pie display in a node.

[FIG. 39]

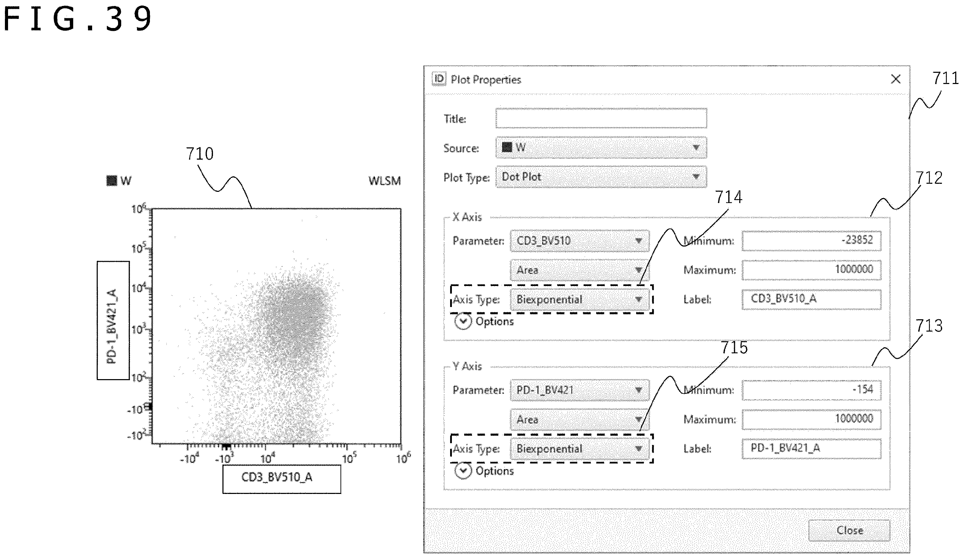

FIG. 39 is a figure depicting an example of two-dimensional plot data and a plot settings window for setting display of the plot data.

[Description of Embodiments]

Suitable modes for carrying out the present disclosure are explained below. Note that embodiments explained below are depicted as representative embodiments of the present disclosure, and the scope of the present disclosure is not limited only to those embodiments. Note that the explanation of the present disclosure is given in the following order.

1. Information Processing System

(1) Explanation of Related Technologies
(2) Overview of Information Processing System
(3) Constituent Elements Included in Information Processing System

(3-1) Server System
(3-2) Data Analysis Client Terminal
(3-3) Data Acquisition Client Terminal
(3-4) Biosample Analyzing Apparatus

(4) Example of Process Flow by Information Processing System

(4-1) Automatic Analysis Process
(4-2) Example of Automatic Analysis Process
(4-3) To-Be-Output Data Acquisition Process
(4-4) Interactive Analysis Process
(4-5) Example of Interactive Analysis Process

(5) Settings of Region
(6) Data Migration
(7) Use of External Storages or Computation Resources
(8) Division of Analysis-Subject Data
(9) Data Sharing
(10) Standardization of To-Be-Output Data
(11) Example 1 of Output Control of Clustering Result Display View
(12) Example 2 of Output Control of Clustering Result Display View
(13) Association between Metaclusters in Clustering Result Display View and Two-Dimensional Plot
(14) Control of Pie Display Axis in Star Chart

2. Information Processing Method

1. Information Processing System

(1) Explanation of Related Technologies

For example, from the perspective of fluorescence measurement optical systems, flow cytometers can be broadly classified into filter-type flow cytometers and spectrum-type flow cytometers. In order to take out only target light information from a target fluorochrome, the filter-type flow cytometers can adopt a configuration like the one depicted in 1 in FIG. 1. Specifically, light generated by light application onto particles is split into multiple beams by wavelength separating means DM such as dichroic mirrors, for example, and the beams are caused to pass through different filters. Then, measurement of the respective beams generated by the splitting is performed by multiple sensors, for example, photomultipliers PMTs or the like. That is, in the filter-type flow cytometers, multicolor fluorescence sensing is performed by performing fluorescence sensing of each wavelength band corresponding to a fluorochrome by using a sensor corresponding to the fluorochrome. At that time, in a case where multiple fluorochromes whose fluorescence wavelengths are proximate to each other are used, a fluorescence correction process can be performed in order to calculate more accurate amounts of fluorescence.

The spectrum-type flow cytometers analyze the amount of fluorescence of each particle by performing, on fluorescence data obtained by sensing of light generated by light application onto particles, deconvolution (Unmixing) with use of spectrum information of a fluorochrome used in staining. As depicted in 2 in FIG. 1, the spectrum-type flow cytometers disperse fluorescence by using a prism dispersive optical element P. In addition, in order to sense the dispersed fluorescence, instead of a large number of light sensors included in the filter-type flow cytometers, the spectrum-type flow cytometers include an array-type sensor, for example, an array-type photomultiplier PMT or the like. The spectrum-type flow cytometers can easily avoid the influence of leakage of fluorescence as compared to the filter-type flow cytometers, and are more suited for an analysis using multiple fluorochromes.

In recent years, in the fields of basic medical sciences and clinical medicine, the increasingly widespread use of multicolor analyses using multiple fluorochromes has been observed in flow cytometry in order to carry out comprehensive interpretation. There is a tendency for the number of fluorochromes used in one multicolor analysis process to increase. If a large number of fluorochromes are used in one-time measurement with a filter-type flow cytometer, as described above, fluorescence from a fluorochrome other than a target fluorochrome leaks into each sensor, and analysis precision lowers. In a case where there is a large number of colors, the problem of leakage of fluorescence can be solved by using a spectrum-type flow cytometer.

An example of an experiment flow using a flow cytometer is explained below with reference to FIG. 2.

The flow of an experiment using a flow cytometer is broadly classified into an experiment planning step ("1: Plan" in FIG. 2) in which cells which are an experiment subject and a method for sensing the cells are examined and an fluorescently-labeled antibody reagent is prepared, a sample preparation step ("2: Preparation" in FIG. 2) in which preparation is performed by actually staining cells such that the cells become suited for measurement, an FCM measurement step ("3: FCM" in FIG. 2) in which the amount of fluorescence of each stained cell is measured with the flow cytometer, and a data analysis step ("4: Data Analysis" in FIG. 2) in which various types of data processing are performed such that desired analysis results are obtained from data recorded in the FCM measurement. Then, these steps can be performed repeatedly as necessary.

In the experiment planning step, first, it is decided which molecules (e.g., antigens, cytokines, or the like) are used as molecules whose expression is used as an indication for determining that there are microparticles (mainly, cells) desired to be sensed by using the flow cytometer. That is, a marker to be used in sensing of microparticles is decided. For example, the decision can be made on the basis of information such as past experimental results or papers. Next, it is examined which fluorochrome is to be used for sensing with the marker. Simultaneously, information such as the number of markers that are desired to be sensed, specifications of an available FCM apparatus, fluorescently-labeled reagents that can be purchased, the spectrum, brightness, price, and delivery date of the fluorochrome is considered comprehensively, and a combination of fluorescently-labeled antibody reagents that are necessary for the actual experiment is decided. This process of deciding a combination of the reagents is typically called a panel design in FCM.

In the sample preparation step, first, the experiment subject is processed to become suited for the FCM measurement. For example, separation and refinement of the cells can be performed. For example, regarding immunocytes or the like derived from blood, red blood cells are removed from the blood by a hemolysis process and density-gradient centrifugation, and white blood cells are extracted. A staining process is performed on a group of the extracted subject cells by using a fluorescently-labeled antibody.

In the FCM measurement step, when microparticles are to be analyzed optically, first, excitation light is emitted from a light source of a light applying section of the flow cytometer, and microparticles flowing in a flow channel are irradiated with the excitation light. Next, fluorescence emitted from the microparticles is sensed by a sensing section of the flow cytometer. Specifically, by using a dichroic mirror, a bandpass filter, or the like, only light with a particular wavelength (target fluorescence) is separated from light emitted from the microparticles, and is sensed by a sensor such as a PMT, for example. At this time, for example, fluorescence is dispersed by using a prism, a diffraction grating, or the like, and, by using a sensor such as a 32-channel PMT or the like, light with different wavelengths is sensed at different channels. As a result, spectrum information regarding sensing light (fluorescence) can easily be obtained.

The flow cytometer can have a functionality of recording fluorescence information regarding each microparticle acquired by the FCM measurement along with information other than the fluorescence information, such as scattered-light information, time information, or positional information. The recording functionality can mainly be executed by a memory or a disk of a computer. Since an analysis of several thousand to several million microparticles is performed under one experiment condition in a typical cell analysis, it is necessary to record a large number of pieces of information in a state where they are organized for each experimental condition.

In the data analysis step, optical intensity data of each wavelength region obtained by the sensing in the FCM measurement step is quantified by using a computer or the like, and the amount of fluorescence (intensity) of each fluorochrome used is determined. This analysis uses a correction method using a reference calculated from experiment data. The reference is calculated by a statistical process by using two types of data which are measurement data of microparticles stained with only one fluorochrome and measurement data of unstained microparticles. The calculated amounts of fluorescence can be recorded on a data recording section included in the computer, along with information such as the names of fluorescence molecules, the measurement dates, or the types of microparticles. The amounts of fluorescence of samples (fluorescence spectral data) estimated by the data analysis are stored, and displayed in a graph according to a purpose, and an analysis of the fluorescence amount distribution of microparticles is performed.

For example, gate setting can often be performed for the analysis of the fluorescence amount distribution, and the ratio of sensing-subject cells in a sample can thereby be calculated. For example, as depicted in FIG. 3, by generating two-dimensional plots related to forward scattered light (FSC) and side scattered light (SSC) and selecting a predetermined range in the plots, the ratios of monocytes and lymphocytes in blood cells included in PBMC can be identified. Further, gate setting and expansion about lymphocytes expressing a predetermined surface marker allow calculation of the ratios of B cells, T cells, and NK cells in the lymphocytes. Moreover, it is also possible to identify the ratio of memory B cells in the B cells, the ratios of killer T cells and helper T cells in the T cells, and, in addition, the ratios of naive T cells and memory T cells in the T cells. For example, as depicted in FIG. 4, it is known that surface markers that respective types of cells express are different among cell types. Accordingly, it is possible to examine cells in a sample by appropriately selecting antibodies that bind to surface markers and a fluorochrome that labels each antibody and then performing an analysis by using a flow cytometer.

An information processing system according to the present disclosure and constituent elements included in the system can be used for an analysis in the data analysis step.

(2) Overview of Information Processing System

For example, information processing in the data analysis step is performed by an information processing apparatus that is attached to a flow cytometer or an information processing apparatus of a user who uses a flow cytometer. However, as described above, in flow cytometry, there is a tendency for the number of fluorochromes used in one-time measurement to increase, and, along with this, there is also a tendency for the information processing amount in the data analysis step to increase. Accordingly, in order to execute the data analysis step in these information processing apparatuses, the information processing apparatuses are required to have higher specifications. This tendency becomes particularly noticeable at a time of execution of what is generally called an Advanced analysis technique such as a dimensional compression process or a clustering process that requires a greater computational load. However, it is often not realistic that these information processing apparatuses have such specifications, and this is not desirable for users as well.

In view of this, it is considered desirable for users if the information processing in the data analysis step can be executed by an analysis system adopting a client-server method, particularly, by an analysis system adopting a client-server method using the cloud.

In addition, as described above, an analysis system adopting a client-server method often requires large operating costs. Moreover, there is a problem related to the processing speed of the analysis system.

The present inventor found that a particular server system can solve at least one of the problems. That is, the present disclosure provides a server system including an automatic analysis processing section that generates to-be-output data by an analysis process on optical intensity data or fluorescence label intensity data acquired by irradiation of a biosample with light, an analysis result data storage section that stores the to-be-output data generated on the basis of the optical intensity data or the fluorescence label intensity data, and an interactive analysis processing section that analyzes the fluorescence label intensity data on the basis of an analysis command for the to-be-output data output to an output apparatus and outputs analysis result data, and an information processing system including the server system.

Since the server system includes the automatic analysis processing section and the interactive analysis processing section, a data analysis step can be executed in the server system. In addition, since the interactive analysis processing section allows execution of an analysis process or adjustment of analysis settings while a user is checking the to-be-output data output on the output apparatus, desired analysis results can easily be acquired.

In addition, since the server system executes processes by the automatic analysis processing section and the interactive analysis processing section only when necessary, the operating costs can also be reduced. In addition, a configuration explained below in the present specification also allows enhancement of the processing speed of the server system.

A configuration example of an information processing system according to the present disclosure is explained below with reference to FIG. 5.

An information processing system 1 depicted in FIG. 5 includes a server system 10, a data analysis client terminal 20, a data acquisition client terminal 30, and a biosample analyzing apparatus 40.

The server system 10 may be connected with the data analysis client terminal 20 and the data acquisition client terminal 30 via a network 50. The network 50 may be a communication network used to perform data transmission/reception. For example, the network 50 may be the Internet, a satellite communication network, a telephone network, or a mobile communication network (e.g., a 4G network, a 5G network, or the like) or may be a combination of these.

For example, the data analysis client terminal 20 and the data acquisition client terminal 30 may be connected to each other by a cable or wirelessly, or may be connected to each other via the network 50.

For example, the bioparticle analyzing apparatus 40 may be connected with the data acquisition client terminal 30 by a cable or wirelessly.

(3) Constituent Elements Included in Information Processing System

Each element included in the information processing system according to the present disclosure is explained below.

(3-1) Server System

The server system 10 is explained with reference to FIG. 6. FIG. 6 is a block diagram depicting an example of a functional configuration of the system. The server system 10 may include an automatic analysis processing section 11, an interactive analysis processing section 12, a to-be-output data generating section 13, a connecting section 14, an optical data storage section 15, an analysis result data storage section 16, and a database 17.

In an embodiment of the present disclosure, the automatic analysis processing section 11 may calculate fluorescence label intensity data from optical intensity data acquired by irradiation of a biosample with light. The optical intensity data may be optical intensity data transmitted to the server system 10 from the data acquisition client terminal 30. The optical intensity data may be stored on the optical data storage section 15, and the automatic analysis processing section 11 can acquire the optical intensity data from the optical data storage section 15.

In another embodiment of the present disclosure, the automatic analysis processing section 11 may not perform the calculation process. That is, the data acquisition client terminal 30 may execute a process of calculating fluorescence label intensity data from optical intensity data, and then, the data acquisition client terminal 30 may transmit the fluorescence label intensity data to the server system 10.

The automatic analysis processing section 11 can execute a process of calculating the fluorescence label intensity data from the optical intensity data. The automatic analysis processing section 11 can execute the calculation process by using analysis settings data retained by the server system 10 in advance. For example, the analysis settings data may be one that is transmitted from the data analysis client terminal 20 or the data acquisition client terminal 30 in advance (particularly, before execution of the calculation process).

The automatic analysis processing section 11 calculates the fluorescence label intensity data by performing a fluorescence correction process or an unmixing process on the optical intensity data, for example. The unmixing process is also called a fluorescence separating process.

Preferably, the automatic analysis processing section 11 executes the unmixing process by using spectral reference data. The spectral reference data to be used in the unmixing process includes spectral data of fluorescence that is generated when a fluorochrome labelling particles is irradiated with predetermined excitation light. The spectral reference data to be used in the unmixing process may include spectral data of fluorescence that is generated when a fluorochrome labelling particles is irradiated with light having a predetermined wavelength and spectral data of fluorescence that is generated when a fluorochrome labelling particles is irradiated with light having a wavelength different from the predetermined wavelength.

The spectral reference data may be stored on any one of the storage sections or the database in the server system 10 in advance, and may be stored on the database 17, for example. Particularly, the spectral reference data may be stored as one piece of metadata described later. The automatic analysis processing section 11 can acquire the spectral reference data from the database 17, for example, and then can execute the unmixing process by using the acquired spectral reference data.

For example, the automatic analysis processing section 11 can perform the unmixing process by using the least square method (LSM), more preferably, the weighted least square method (WLSM). For example, the unmixing process using the least square method may be performed by using a fluorescence intensity correction method described in Japanese Patent No. 5985140. For example, the fluorescence intensity correction method can be performed by using the following mathematical formula (1) for WLSM.
[Math. 1]

$$\begin{bmatrix} x_1 \\ \vdots \\ x_n \end{bmatrix} = ([S^T][L][S])^{-1}[S^T][L]\begin{bmatrix} y_1 \\ \vdots \\ y_m \end{bmatrix} \quad \cdots (1)$$

$$L = \begin{bmatrix} \lambda_1 & 0 & 0 \\ 0 & \ddots & 0 \\ 0 & 0 & \lambda_m \end{bmatrix}, \quad \lambda_i = \frac{1}{max(y_i, \ 0) + offset},$$

In mathematical formula (1) described above, $x_n$ denotes the fluorescence intensity of an n-th fluorochrome, $[S^T]$ denotes the transpose of a spectral reference, $[L]$ denotes a denotes a weighting matrix, $[S]$ denotes the matrix of the spectral reference, $y_i$ denotes a measurement value of an i-th light sensor, $\lambda_i$ denotes a weight of the i-th light sensor, max $(y_i,0)$ denotes a value which is the larger one of a sensing value of the i-th sensor and zero, and offset' denotes a value decided on the basis of a sensing value of each sensor.

The fluorescence wavelength distribution of fluorochromes is wide in some cases. Accordingly, for example, a PMT used for sensing fluorescence generated from a certain fluorochrome can also sense fluorescence generated from another fluorochrome. That is, optical data acquired by each PMT can be data in which fluorescence data from multiple fluorochromes is superimposed. In view of this, correction for separating the optical data into fluorescence data from each fluorochrome is necessary. The unmixing process is a technique for the correction, the unmixing process separates data in which fluorescence label intensity data from multiple fluorochromes is superimposed into fluorescence label intensity data from each fluorochrome, and fluorescence label intensity data from each fluorochrome is obtained.

The automatic analysis processing section 11 performs an analysis process on the fluorescence label intensity data. The automatic analysis processing section 11 generates analysis result data by the analysis process. To-be-output data can be generated from the analysis result data. The to-be-output data is transmitted to the data analysis client terminal 20, and then, the data analysis client terminal 20 causes an output apparatus to output the to-be-output data. For example, the output apparatus may be a display apparatus. The output apparatus can be configured to allow a user to input an analysis command described later.

Preferably, in response to storage of the optical intensity data on the optical data storage section 15, the automatic analysis processing section 11 executes a process of calculating the fluorescence label intensity data from the optical intensity data. Then, the automatic analysis processing section 11 can analyze the fluorescence label intensity data, and generate the analysis result data. Further, the automatic analysis processing section 11 can generate the to-be-output data from the analysis result data. For example, in response to storage of the optical intensity data on the optical data storage section 15, the automatic analysis processing section 11 may start the automatic analysis process. That is, the automatic analysis processing section 11 may execute an event-driven analysis process triggered by the storage.

In response to storage of the optical intensity data on the optical data storage section 15, the automatic analysis processing section 11 reserves computation resources for the automatic analysis process. That is, the automatic analysis processing section 11 can handle the storage as reception of an analysis start command, and, in response to the reception, reserve the computation in the server system 10. By using the reserved computation resources, the automatic analysis processing section 11 can execute the fluorescence label intensity data calculation process, the fluorescence label intensity data analysis process, and the to-be-output data generation process using the analysis result data.

On the basis of the analysis command for the to-be-output data output on the output apparatus, the interactive analysis processing section 12 can analyze the fluorescence label intensity data, and generate analysis result data. The to-be-output data may be to-be-output data generated by the automatic analysis processing section 11 or may be to-be-output data acquired or generated by the to-be-output data generating section 13 described later.

The interactive analysis processing section 12 may execute a process of calculating the fluorescence label intensity data from the optical intensity data. Then, the interactive analysis processing section 12 may analyze the calculated fluorescence label intensity data on the basis of the analysis command, and generate the analysis result data. Then, the interactive analysis processing section 12 can generate to-be-output data from the generated analysis result data.

In response to reception of an analysis start command, the interactive analysis processing section 12 may reserve computation resources for processes by the interactive analysis processing section 12. After the reservation, the interactive analysis processing section 12 waits until an analysis command is transmitted from the data analysis client terminal 20.

As described above, the automatic analysis processing section 11 can reserve computation resources for processes by the automatic analysis processing section in response to reception of the analysis start command, and also the interactive analysis processing section 12 can reserve computation resources for processes by the interactive analysis processing section 12 in response to reception of the analysis start command. Accordingly, in the present disclosure, processes by the automatic analysis processing section and processes by the interactive analysis processing section may be executed on mutually different computation resources.

In response to reception of the analysis command transmitted from the data analysis client terminal 20, the interactive analysis processing section 12 can execute the fluorescence label intensity data calculation process, the fluorescence label intensity data analysis process, and the to-be-output data generation process using the analysis result data. These processes may be event-driven analysis processes triggered by reception of the analysis command. That is, an interactive analysis process according to the present technology may be an event-driven analysis process.

The interactive analysis processing section 12 may execute the fluorescence label intensity data calculation process, the fluorescence label intensity data analysis process, and the to-be-output data generation process using the analysis result data, similarly to these processes by the automatic analysis processing section 11.

The connecting section 14 is a functional element for executing an RPC (Remote Procedure Call) in an interactive process between the server system 10 (particularly, the interactive analysis processing section 12) and the data analysis client terminal 20. For example, the connecting section 14 causes the server system 10 to execute an analysis command input on the data analysis client terminal 20.

The to-be-output data generating section 13 can generate to-be-output data from analysis result data stored on the analysis result data storage section 16. Then, the to-be-output data generating section 13 transmits the to-be-output data to the data analysis client terminal 20. The amount of computation resources required for processes by the to-be-output data generating section 13 is small. Accordingly, the operating cost of the server system can be reduced.

Processes by the to-be-output data generating section 13 may be executed by a virtual server that is active at any time in the server system 10. Since the virtual server is active at any time, a to-be-output data acquisition process can be executed at a high speed without waiting time that accompanies server activation.

In addition, the to-be-output data generating section 13 may be configured as a serverless architecture.

The optical data storage section 15 stores optical intensity data and/or fluorescence label intensity data. The optical data storage section 15 may include two storage sections which are an optical intensity data storage section that stores optical intensity data and a fluorescence label intensity data storage section that stores fluorescence label intensity data.

The analysis result data storage section 16 stores analysis result data generated by the automatic analysis processing section 11 on the basis of fluorescence label intensity data and/or analysis result data generated by the interactive analysis processing section 12 on the basis of fluorescence label intensity data. Further, the analysis result data storage section 16 stores to-be-output data generated from these pieces of the analysis result data.

The database 17 may store various types of metadata. The metadata may include analysis settings data. Particularly, the database 17 may store analysis settings data to be used in processes by the automatic analysis processing section and/or processes by the interactive analysis processing section.

For example, the analysis settings data may include additional data to be referred to or used in a process of analyzing optical intensity data. For example, the additional data can include data related to a biosample itself and/or data related to biosample analysis settings.

In addition, for example, the analysis settings data can include data (e.g., data including spectral reference data) to be used for calculating fluorescence label intensity data from optical intensity data and/or data (e.g., data including an analysis command) to be used in a fluorescence intensity labeled data analysis process.

Examples of configurations of the metadata are explained with reference to FIGs. 7 and 8.

As depicted in FIG. 7, project data (project in FIG. 7) included in the metadata may be one that identifies a project unit set as desired by a user, for example.

The metadata may include one or more of project data, preset fluorochrome data, fluorochrome data set as desired, spectral reference data, autofluorescence data, and instrument settings data.

For example, one piece of project data may be associated with one or more pieces of data in the preset fluorochrome data (Fluorochrome (preset) in FIG. 7), fluorochrome data (Fluorochrome (custom)) set as desired, spectral reference data (Spectral Reference), autofluorescence data (Autofluorescence), and instrument settings data (Instrument Settings).

The preset fluorochrome data includes data (e.g., fluorochrome name data, etc.) related to one or more fluorochromes used in common for data analysis for which the project data is used.

The fluorochrome data set as desired includes data (e.g., fluorochrome name data, etc.) related to a fluorochrome selected as desired by a user, for example, for each experiment or for each sample in the data analysis for which the project data is used.

The spectral reference data includes spectral reference data of each fluorochrome included in the preset fluorochrome data and the fluorochrome data set as desired.

The autofluorescence data includes data related to autofluorescence of a biosample that is analyzed by using the project data.

The instrument settings data includes data related to analysis settings of a biosample analyzing apparatus that has acquired optical intensity data of the biosample that is analyzed by using the project data.

The number of pieces of the data associated with one piece of project data may be one or greater than one. For example, one piece of project data may be associated with one piece of or two or more pieces of preset fluorochrome data.

The metadata may include experiment data. For example, one piece of project data may be associated with one piece of or two or more pieces of experiment data (Experiment in FIG. 7). For example, each piece of experiment data may include one or more of experiment name data, data regarding the name of a user who has created the experiment data, and data regarding the date/time when the experiment data has been created.

The metadata may include plate data. For example, one piece of experiment data may be associated with one piece of or two or more pieces of plate data (plate in FIG. 7). For example, each piece of plate data includes data that identifies a plate (particularly, a well plate or a microtiter plate) which is the subject of an analysis by a biosample analyzing apparatus, and may include one or more of a plate name, data regarding the name of a user who has created the plate data, data regarding the date/time when the plate data has been created, and data regarding the type of the plate, for example.

The metadata may include sample group data. For example, one piece of plate data may be associated with one piece of or two or more pieces of sample group data (Sample Group in FIG. 7). For example, each piece of sample group data includes data that identifies a group of biosamples included in each piece of plate data, and may include one or more of sample group name data, data regarding the name of a user who has created the sample group data, and data regarding the date/time when the sample group data has been created, for example.

The metadata may include protocol data.

For example, each piece of sample group data may be associated with protocol data (Protocol in FIG. 7). For example, multiple pieces of sample group data may be associated with the same protocol data or may be associated with mutually different pieces of protocol data.

For example, each piece of protocol data includes data that identifies an analysis protocol of a biosample, and may include one or more of unmixing configuration data (Unmixing Config), fluorochrome settings data (Color Palette), instrument settings data (Measurement Settings), and shared worksheet settings data (Shared Worksheet), for example.

The unmixing configuration data may include data that identifies a manner of an unmixing process (e.g., an unmixing matrix configuration, computation techniques, or the like).

The fluorochrome settings data may include data related to allocation of a fluorochrome to biomolecules, and may include results of panel designing, for example.

The instrument settings data may include data related to settings of an analysis by a biosample analyzing apparatus.

The worksheet settings data may include data related to analysis settings to be applied in common to all biosamples included in one piece of sample group data. For example, the worksheet settings data may include gate settings data to be applied in common to all biosamples included in one piece of sample group data.

One piece of sample group data may be associated with one piece of or two or more pieces of sample data (Sample in FIG. 7). For example, each piece of sample data may include one or more of individual worksheet settings data (Individual Worksheet) to be applied to an analysis of each sample, unmixing configuration data (Sample Unmixing Config) to be applied to an analysis of each sample, a data path (Raw Data) to optical intensity data obtained by measurement of each sample, and fluorescence label intensity data (Unmixed Data) calculated from optical intensity data obtained by measurement of each sample.

The metadata may include comparison worksheet data. For example, one piece of experiment data may be associated with one piece of or two or more pieces of comparison worksheet data (Comparison Worksheet in FIG. 7). The comparison worksheet data may be worksheet data to be used for comparison of analysis results about each piece of sample data or for superimposed display of the analysis results.

The worksheet settings data in the data depicted in FIG. 7 is explained below in more detail with reference to FIG. 8.

As depicted in FIG. 8, the worksheet settings data may include one, two, three, four, or five pieces of data among region settings data (Region in FIG. 8), gate settings data (Gate), axis parameter settings data (Axis Parameter), plot settings data (Plot), axis tick settings data (Axis Tick setting), and statistic settings data (Statistic), or may include all the six pieces of data.

The region settings data is data related to a region set by a gate in a plot displayed on a worksheet, and, for example, includes data related to a range of fluorescence label intensity and/or a range of wavelengths specified by the gate.

For example, the gate settings data includes data related to the type of gate settings in a plot displayed on a worksheet and/or data related to gate parameters. For example, the data related to the type of gate settings includes data related to the shape of a gate, and the shape may be a rectangle, a line, a polygon, an ellipse, a four-quadrant, or the like, for example. For example, the data related to the gate parameters may include data related to the position and/or size of a gate.

For example, the axis parameter settings data may include data related to the type of a field of a plot displayed on a worksheet and/or data related to the type of a signal adopted for the plot. For example, the type of a field may be forward scattered light (FSC), side scattered light (SSC), fluorescence, a wavelength, a channel (the type or number of optical sensing channels), the number of events, optical intensity, or the like. Any of these types of field may be adopted as an axis parameter. In addition, for example, the data related to the type of a signal may be data related to an area size, a height, or a width.

The plot settings data may include data related to the type of a plot displayed on a worksheet. For example, the type of a plot may be a spectral plot, a histogram plot, a dot plot, a density plot, or the like.

The axis tick settings data may include data related to ticks of an axis of a plot displayed on a worksheet. For example, the data may include one or more of data related to the scale of ticks, data related to the maximum value and/or minimum value of ticks, and data related to whether settings are associated with a biexponential.

For example, the statistic settings data may include data specifying statistics output on a worksheet. For example, the statistics may be one or more of the number of events, the ratio (parent%) of the number of events in the gate to the number of events in a parent gate, the ratio (total!) of the number of events in the gate to the number of all events of a relevant sample, the average, the maximum value, the minimum value, the standard deviation, the coefficient of variation (CV), and the median.

FIG. 9 depicts a hardware configuration example of a server apparatus included in the server system 10. The server system 10 may have the multiple server apparatuses. In addition, the multiple server apparatuses may be present in one data center or may be arranged dispersedly in multiple data centers that are present at mutually different locations or in mutually different countries.

A server apparatus 1000 depicted in FIG. 9 includes a CPU (Central Processing Unit) 1001, a RAM 1002, and a ROM 1003. The CPU 1001, the RAM 1002, and the ROM 1003 are interconnected via a bus 1004. The bus 1004 is further connected with an input/output interface 1005.

The input/output interface 1005 is connected with a communication apparatus 1006, a storage apparatus 1007, a drive 1008, an output section 1009, and an input section 1010.

The communication apparatus 1006 connects the server apparatus 1000 to a network 1011 by a cable or wirelessly. The communication apparatus 1006 allows the server apparatus 1000 to acquire various types of data (e.g., image data, etc.) via the network 1011. For example, the acquired data can be stored on the storage apparatus 1007. The type of the communication apparatus 1006 may be selected as appropriate by those skilled in the art.

The storage apparatus 1007 can store an operating system, a program for causing the server system to realize an information processing method according to the present disclosure, and other various types of programs, as well as image data, various types of data to be used in the information processing method according to the present disclosure, and other various types of data. For example, the operating system may be a UNIX (registered trademark) OS, particularly, LINUX (registered trademark), or a WINDOWS (registered trademark) OS.

The drive 1008 can read out data recorded on a recording medium (e.g., optical intensity data, fluorescence label intensity data, analysis result data, to-be-output data, or the like) or programs, and output them to a RAM 1003. For example, the recording medium is an HDD, an SSD, a micro SD memory card, an SD memory card, or a flash memory, but is not limited to these.

The output section 1009 may be connected with an output apparatus, for example, a display apparatus. The input section 1010 can accept input for operating the server system itself.

(3-2) Data Analysis Client Terminal

The data analysis client terminal 20 is explained with reference to FIG. 10. FIG. 10 is a block diagram depicting an example of a functional configuration of the terminal. The data analysis client terminal 20 includes a processing section 21, an analysis instructing section 22, a connecting section 23, a to-be-output data storage section 24, and a communication section 25.

The processing section 21 performs a process of causing the output apparatus attached to the data analysis client terminal 20 to output to-be-output data transmitted from the server system 10. Particularly, the to-be-output data is displayed on a window on a screen displayed on the output apparatus (particularly, the display apparatus). That is, the data analysis client terminal may include an output apparatus on which the to-be-output data is output.

The analysis instructing section 22 transmits, to the server system 10, a start request for the interactive analysis process explained above.

In addition, the analysis instructing section 22 accepts input of an analysis command to be used in the interactive analysis process explained above, and then transmits the analysis command to the server system 10. The analysis command may include the worksheet settings data explained above. The interactive analysis processing section 12 can execute the analysis process by referring to the worksheet settings data, and to-be-output data according to the worksheet settings data can thereby be generated. In such a manner, the data analysis client terminal according to the present disclosure may be configured to transmit, to the server system, the analysis command for the to-be-output data output on the output apparatus. In addition, the data analysis client terminal according to the present disclosure may be configured to cause the output apparatus to output a window on which the to-be-output data is displayed, and accept input of the analysis command on the window.

The connecting section 23 is a functional element for executing an RPC (Remote Procedure Call) in an interactive process between the server system 10 (particularly, the interactive analysis processing section 12) and the data analysis client terminal 20 (particularly, the analysis instructing section 22). For example, the combination of the connecting section 23 of the data analysis client terminal 20 and the connecting section 14 of the server system 10 may allow transmission of the analysis command input on the data analysis client terminal 20 to the server system 10 and subsequent execution of the analysis command in the server system 10.

The to-be-output data storage section 24 stores to-be-output data transmitted from the server system 10.

The communication section 25 receives, from the server system 10, to-be-output data created by the server system on the basis of fluorescence label intensity data.

FIG. 11 depicts a hardware configuration example of the data analysis client terminal 20. Note that, for example, the terminal may be a general-purpose information processing apparatus (particularly, a computer).

An information processing apparatus 1100 depicted in FIG. 11 includes a CPU (Central Processing Unit) 1101, a RAM 1102, and a ROM 1103. The CPU 1101, the RAM 1102, and the ROM 1103 are interconnected via the bus 1004. The bus 1104 is further connected with an input/output interface 1105.

The input/output interface 1105 is connected with a communication apparatus 1106, a storage apparatus 1107, a drive 1108, an output section 1109, and an input section 1110.

The communication apparatus 1106 connects the information processing apparatus 1100 to a network 1111 by a cable or wirelessly. The communication apparatus 1106 allows the information processing apparatus 1100 to transmit or receive various types of data via the network 1111. For example, by the communication apparatus 1106, transmission of various types of data to the server system 10 or reception of various types of data from the server system 10 is performed. The type of the communication apparatus 1106 may be selected as appropriate by those skilled in the art.

The storage apparatus 1107 can store an operating system, a program for causing a to-be-output data output section to output to-be-output data, a program for realizing an interactive analysis process, and other various types of programs, as well as various types of data to be used in the information processing according to the present disclosure and other various types of data. For example, the operating system may be a UNIX (registered trademark) OS, particularly, LINUX (registered trademark), or a WINDOWS (registered trademark) OS.

The drive 1108 can read out data recorded on a recording medium (e.g., to-be-output data, etc.) or programs, and output them to a RAM 1103. For example, the recording medium is an HDD, an SSD, a micro SD memory card, an SD memory card, or a flash memory, but is not limited to these.

The output section 1109 causes an output apparatus to output to-be-output data. For example, the output apparatus may be a display apparatus. For example, the input section 1110 accepts input of an analysis command in an interactive analysis process. For example, the input section 1110 may be connected with an input apparatus such as a keyboard or a mouse, and input of the analysis command can be performed by using these input apparatuses.

(3-3) Data Acquisition Client Terminal

The data acquisition client terminal 30 is explained with reference to FIG. 12. FIG. 12 is a block diagram depicting an example of a functional configuration of the terminal. The data acquisition client terminal 30 includes a data acquiring section 31, a transmitting section 32, a data processing section 33, and a data storage section 34.

The data acquiring section 31 acquires measurement data transmitted from the biosample analyzing apparatus 40. The measurement data includes optical intensity data that is the subject of processes by the automatic analysis processing section 11. The optical intensity data may be optical intensity data acquired by irradiation of a biosample with light.

The transmitting section 32 transmits, to the server system 10 (particularly, the optical data storage section 15 of the server system 10) measurement data (including optical intensity data) acquired by the data acquiring section 31 or measurement data that has been subjected to processes, by the data processing section 33, to be described later. In addition to the measurement data, the transmitting section 32 can also transmit analysis settings data to the server system 10. Preferably, in response to acquisition of the optical intensity data, the transmitting section 32 transmits the optical intensity data or the optical intensity data and the analysis settings data to the server system. For example, in response to acquisition of the measurement data, the transmitting section 32 may automatically start transmission of the measurement data or the measurement data that has been processed (and the analysis settings data). In such a manner, the data client terminal according to the present disclosure may transmit the optical intensity data to the server system 10. Further, the data acquisition client terminal according to the present disclosure may be configured to transmit the optical intensity data (and the analysis settings data) to the server system 10 in response to acquisition of the optical intensity data.

In addition, in an embodiment of the present disclosure, the data acquisition client terminal 30 may execute a calculation process of calculating the fluorescence label intensity data from the optical intensity data. The calculation process may be executed as explained in (3-1) above, and, for example, may be a fluorescence correction process or an unmixing process. In this embodiment, the transmitting section 32 can transmit the fluorescence label intensity data (or the fluorescence label intensity data that has been subjected to processes, by the data processing section 33, to be described later) to the server system 10 (particularly, the optical data storage section 15 of the server system 10). In such a manner, the data client terminal according to the present disclosure may transmit the fluorescence label intensity data to the server system 10. Further, the data acquisition client terminal according to the present disclosure may be configured to transmit the fluorescence label intensity data to the server system 10 in response to acquisition of the fluorescence label intensity data.

In addition, in the present disclosure, the transmitting section 32 may transmit both the measurement data and the fluorescence label intensity data to the server system 10.

The data acquisition client terminal 30 has the transmitting section 32 as a functional unit separately from the data acquiring section 31, and can thereby execute a data acquisition process and an uploading process separately. As a result, without exerting an influence on the process of data acquisition from the biosample analyzing apparatus 40, it is possible to upload measurement data (including optical intensity data) or fluorescence label intensity data calculated from the measurement data or both types of the data to the server system 10.

The data processing section 33 can execute a predetermined process on the measurement data and/or the fluorescence label intensity data acquired by the data acquiring section 31. The process may be a compression process, a data processing process (e.g., a format conversion process), or the like. The data processing process can convert the measurement data and/or the fluorescence label intensity data into a format suited for information processing in the server system 10, and makes processes in the server system 10 efficient. In such a manner, the data acquisition client terminal according to the present disclosure may be configured to perform a predetermined process on the optical intensity data or the fluorescence label intensity data in response to acquisition of the optical intensity data or the fluorescence label intensity data, and then transmit the processed optical intensity data or fluorescence label intensity data to the server system.

The data storage section 34 can store the measurement data or the measurement data that has been processed. The data storage section 34 may store the fluorescence label intensity data or the fluorescence label intensity data that has been processed.

The contents explained about the data analysis client terminal 20 in (3-2) above holds true of a hardware configuration example of the data acquisition client terminal 30. Note that, for example, the data acquisition client terminal 30 may also be a general-purpose information processing apparatus (particularly, a computer).

(3-4) Biosample Analyzing Apparatus

For example, the biosample analyzing apparatus may be a flow cytometer as described above, but is not limited to this. A configuration example of the biosample analyzing apparatus is depicted in FIG. 13. As depicted in FIG. 13, the biosample analyzing apparatus 40 includes a light applying section 101 that irradiates a biosample S flowing through a flow channel C with light, a sensing section 102 that senses light generated by the irradiation, and an information processing section 103 that processes information regarding the light sensed by the sensing section. For example, examples of the biosample analyzing apparatus 40 include a flow cytometer and an imaging cytometer. The biosample analyzing apparatus 40 may include an isolating section 104 that isolates particular bioparticles P in the biosample. For example, examples of the biosample analyzing apparatus 40 including the isolating section include a cell sorter.

(Biosample)

The biosample S may be a liquid sample containing bioparticles. For example, the bioparticles are cells or acellular bioparticles. The cells may be living cells, and more specific examples include blood cells such as red blood cells or white blood cells and reproductive cells such as sperm cells or fertilized eggs. In addition, the cells may be ones directly collected from a specimen such as whole blood or may be cultured cells acquired after being cultured. Examples of the acellular bioparticles include extracellular vesicles, particularly, exosome vesicles, microvesicles, and the like. The bioparticles may be labeled by one or more labeling substances (e.g., dyes (particularly, fluorochromes), fluorochrome-labeled antibodies, and the like). Note that the biosample analyzing apparatus of the present disclosure may analyze particles other than bioparticles, and may analyze beads or the like for calibration or the like.

(Flow Channel)

The flow channel C can be configured such that the biosample flows therethrough, particularly, such that the flow of a stream of approximately one line of bioparticles contained in the biosample is formed. A flow channel structure including the flow channel C may be designed such that a laminar flow is formed, and particularly, is designed such that a laminar flow in which the flow of the biosample (sample flow) is surrounded by the flow of a sheath liquid is formed. The design of the flow channel structure may be selected as appropriate by those skilled in the art, or a known design may be adopted. The flow channel C may be formed in a flow channel structure (particularly, a flow channel structure in which focusing is performed) such as a microchip (a chip having a micrometer-order flow channel) or a flow cell. The width of the flow channel C is equal to or smaller than 1 mm, and particularly, may be equal to or greater than 10 $\mu$m

and equal to or smaller than 1 mm. The flow channel C and the flow channel structure including the flow channel C may include a material such as plastic or glass.

The apparatus according to the present disclosure may be configured such that the biosample flowing in the flow channel C, particularly, bioparticles in the biosample, is irradiated with light from the light applying section. The apparatus according to the present disclosure may be configured such that an irradiation point (interrogation point) of light on the biosample is located in the flow channel structure in which the flow channel C is formed or may be configured such that the irradiation point of the light is located outside the flow channel structure. Examples of the former include a configuration in which the flow channel C in a microchip or a flow cell is irradiated with the light. In the latter case, bioparticles after exiting the flow channel structure (particularly, its nozzle section) may be irradiated with the light, and, for example, examples thereof include a flow cytometer adopting Jet in Air method.

(Light Applying Section)

The light applying section 101 includes a light source section that emits light and a light guide optical system that guides the light to the flow channel C. The light source section includes one or more light sources. For example, the type of the light sources can be laser beam sources or LEDs. The wavelength of light emitted from each light source may be the wavelength of any of ultraviolet light, visible light, and infrared light. For example, the light guide optical system includes optical components such as a beam splitter group, a mirror group, or an optical fiber. In addition, the light guide optical system may include a lens group for condensing light, and can include an objective lens, for example. There may be one or more irradiation points of light on the biosample. The light applying section 101 may be configured to condense light applied from one light source or multiple different light sources onto one irradiation point.

(Sensing Section)

The sensing section 102 includes at least one light sensor that senses light generated by light application onto particles by the light applying section. For example, the light to be sensed is fluorescence or scattered light (e.g., any one or more of forward scattered light, backward scattered light, and side scattered light). Each light sensor includes one or more light receiving elements, and has a light receiving element array, for example. As a light receiving element or light receiving elements, each light sensor may include one or more PMTs (photomultipliers) and/or photodiodes such as APDs or MPPCs. For example, each of the light sensors includes a PMT array including multiple PMTs that are arrayed one-dimensionally. In addition, the sensing section may include an imaging element such as a CCD or a CMOS. By using the imaging element, the sensing section can acquire an image (e.g., a bright field image, a dark a field image, a fluorescence image, and the like) of bioparticles.

The sensing section includes a sensing optical system that causes light with a predetermined sensing wavelength to reach a corresponding light sensor. The sensing optical system includes dispersing sections such as prisms or diffraction gratings or wavelength separating sections such as dichroic mirrors or optical filters. For example, the sensing optical system may be configured to disperse light from bioparticles, and cause multiple light sensors the number of which is greater than the number of fluorochromes to sense light in different wavelength bands. A flow cytometer including such a sensing optical system is called a spectrum-type flow cytometer. In addition, for example, the sensing optical system may be configured to separate light corresponding to the fluorescence wavelength band of a fluorochrome from light from bioparticles, and cause a corresponding light sensor to sense the separated light.

In addition, the sensing section can include a signal processing section that converts electric signals obtained by the light sensor into digital signals. The signal processing section may include an A/D converter as an apparatus to perform the conversion. The digital signals obtained by the conversion by the signal processing section can be transmitted to the information processing section. The digital signals can be handled by the information processing section as data related to light (hereinafter, also referred to as "optical data"). For example, the optical data may be optical data including fluorescence data. More specifically, the optical data may be optical intensity data, and the optical intensity may be optical intensity data of light including fluorescence (feature quantities such as Area, Height, or Width may be included).

(Information Processing Section)

For example, the information processing section 103 includes a processing section that executes processes of various types of data (e.g., optical data) and a storage section that stores various types of data.

In a case where the biosample analyzing apparatus includes an isolating section described later, the information processing section can execute a determination as to whether to isolate bioparticles, on the basis of optical data and/or mode information. Then, on the basis of a result of the determination, the information processing section can control the isolating section, and isolation of the bioparticles by the isolating section can be performed.

The information processing section may be configured as a general-purpose computer, and, for example, may be configured as an information processing apparatus including a CPU, a RAM, and a ROM. The information processing section may be included in a housing including the light applying section and the sensing section, or may be located outside the housing. For example, the information processing section may be realized by the data acquisition client terminal 30.

(Isolating Section)

For example, according to the result of the determination by the information processing section, the isolating section 104 can execute isolation of bioparticles. The isolation method may be a method in which droplets containing bioparticles are generated by vibration, an electric charge is applied to the isolation-subject droplets, and the advancing direction of the droplets is controlled by an electrode. The isolation method may be a method in which bioparticles are isolated by controlling the advancing direction of bioparticles in the flow channel structure. For example, the flow channel structure is provided with a control mechanism that uses pressure (injection or suction) or an electric charge. Examples of the flow channel structure include a chip (e.g., a chip described in 2020-76736) in which the flow channel C has, at its downstream portion, a flow channel structure that branches into a recovery flow channel and a drainage flow channel and in which particular bioparticles flow to and are recovered by the recovery flow channel.

For example, the biosample analyzing apparatus 40 may be a microscope apparatus for executing multicolor fluorescence imaging or the like, particularly, a fluorescence microscope apparatus. In recent years, also in fluorescence imaging, there is a tendency for the number of used fluorescent bodies to increase, and the information processing system according to the present disclosure may be executed on optical intensity data acquired by the microscope apparatus.

(4) Example of Process Flow by Information Processing System

An information processing method executed by the information processing system 1 may include an automatic analysis process step. Further, in addition to the automatic analysis process step, the information processing method may include an interactive analysis process step of using to-be-output data generated by the automatic analysis process step.

In addition, the information processing method executed by the information processing system 1 may include a to-be-output data acquisition step of acquiring to-be-output data based on analysis result data present in the server system and an interactive analysis process step of using the to-be-output data obtained by the acquisition process.

The automatic analysis process, the to-be-output data acquisition process, and the interactive analysis process are explained below.

(4-1) Automatic Analysis Process

The flow of the automatic analysis process by the information processing system 1 is explained with reference to FIG. 14.

In Step S101, the data acquisition client terminal 30 acquires optical intensity data of a biosample from the biosample analyzing apparatus 40. The optical intensity data may be optical intensity data acquired by irradiation of the biosample with light.

In Step S101, in addition to the optical intensity data, the data acquisition client terminal 30 may acquire additional data to be referred to or used in a process of analyzing the optical intensity data. For example, the additional data can include data related to the biosample itself and/or data related to analysis settings about the biosample. Examples of the data related to the biosample itself include an attribute of the biosample (e.g., the type of a creature from which the biosample is derived, the type of a body fluid from which the biosample is derived, the type of an organ from which the biosample is derived, the type of a disorder, or the like), a fluorochrome labelling the biosample, the creator and date of creation of the biosample, and the like, for example. Examples of the data related to the analysis settings about the biosample include settings of the analyzing apparatus, an unmixing configuration, measurement conditions, and the like, for example, but are not limited to these.

Note that, in Step S101, the data acquisition client terminal 30 may execute a process of calculating fluorescence label intensity data from the optical intensity data.

In Step S102, the data acquisition client terminal 30 transmits the optical intensity data to the server system 10. In Step S102, in addition to the optical intensity data, the data acquisition client terminal 30 can transmit the additional data.

Note that, in a case where the process of calculating the fluorescence label intensity data is executed in Step S101, in Step S102, the data acquisition client terminal 30 can transmit the fluorescence label intensity data to the server system 10.

Preferably, in order to automatically execute the series of steps in (4-1), the data acquisition client terminal 30 may execute the transmission in response to acquisition of the optical intensity data or the fluorescence label intensity data from the biosample analyzing apparatus 40 in Step S101. More specifically, by being triggered by the start of reception of the optical intensity data, the data acquisition client terminal 30 may start the transmission of the optical intensity data to the server system 10. The transmission may be performed via the network 50. Note that, although the data acquisition client terminal 30 may start the transmission of the optical intensity data to the server system 10 by being triggered by completion of the reception of the optical intensity data, as described above, completion of the uploading can be made earlier by treating the start of the transmission of the optical intensity data as a trigger.

In Step S103, the server system 10 receives, via the network 50, the optical intensity data or the fluorescence label intensity data transmitted from the data acquisition client terminal 30. The server system 10 stores the optical intensity data or the fluorescence label intensity data on the optical data storage section 15.

In Step S104, the server system 10 executes an automatic analysis process on the optical intensity data or the fluorescence label intensity data. The automatic analysis process is executed particularly by the automatic analysis processing section 11. In the automatic analysis process, the server system 10 can calculate fluorescence label intensity data from the optical intensity data, and then perform an analysis process on the fluorescence label intensity data to generate to-be-output data. In addition, in a case where the server system 10 has received the fluorescence label intensity data, in the automatic analysis process, the server system 10 can execute an analysis process on the received fluorescence label intensity data without performing the process of calculating the fluorescence label intensity data.

Details of the process in Step S104 are explained below with reference to FIG. 15.

In Step S151 depicted in FIG. 15, the automatic analysis processing section 11 starts the automatic analysis process. Preferably, the automatic analysis process is started in response to storage of the optical intensity data (or the fluorescence label intensity data) on the optical data storage section 15 in Step S103. Particularly, the automatic analysis process may be an event-driven analysis process triggered by the storage.

For example, the process by the automatic analysis processing section 11 may be executed in a serverless manner, and the automatic analysis processing section 11 may be configured as what is generally called a serverless architecture.

Note that the "process executed in a serverless manner" in the present specification may mean not a process that does not use a server, but mean that only predetermined information processing is executed in an event-driven format on a server architecture having been constructed in advance, that is, the information processing is executed in units of functions. The server architecture having been constructed may be one that has been constructed in advance by a business operator that provides analysis services to a user of the data analysis client terminal 20 by the server system 10, and may be one that has been constructed by what is generally called a cloud business operator.

In a case where the server system 10 is Amazon Web Services (trademark), for example, the process by the automatic analysis processing section 11 may be executed by AWS Lambda, for example, and is particularly executed by a system including the combination of AWS Lambda and Amazon EC2.

In Step S152, the automatic analysis processing section 11 reserves, on the server system 10, computation resources for executing a process of analyzing the optical intensity data (or the fluorescence label intensity data). The computation resources function as a virtual server for executing the analysis process. In Step S152, the virtual server is activated.

In Step S153, the automatic analysis processing section 11 (particularly, the virtual server) downloads the optical intensity data (or the fluorescence label intensity data) stored on the optical data storage section 15.

In Step S154, the automatic analysis processing section 11 (particularly, the virtual server) acquires data necessary for an analysis. For example, the data necessary for the analysis can include data to be used for calculation of fluorescence label intensity data and data to be used in the fluorescence intensity labeled data analysis process. These pieces of data may be stored on any one of the storage sections or the database in the server system 10, and may be stored on the database 17, for example.

The data to be used for calculation of fluorescence label intensity data can include spectral reference data, for example. The data is used for an unmixing process.

The data to be used in the fluorescence intensity labeled data analysis process can further include an analysis command. For example, the analysis command includes a gate settings command, a plot settings command, an axis settings command, an axis display settings command, a statistics settings command, and a region settings command. For example, the analysis command is used for obtaining analysis result data such as a two-dimensional plot that the user desires to obtain. In addition, the analysis command may include a clustering command.

The analysis command may be one that has been transmitted to the server system 10 from the data analysis client terminal 20 or the data acquisition client terminal 30 in advance.

In Step S155, the automatic analysis processing section 11 (particularly, the virtual server) can execute a process of calculating fluorescence label intensity data from the optical intensity data. Then, the automatic analysis processing section 11 executes a process of generating analysis result data by using the calculated fluorescence label intensity data. Note that, in a case where the server system 10 has received the fluorescence label intensity data in Step S103, the calculation process may be omitted.

The process of calculating the fluorescence label intensity data may be executed by using data to be used for the calculation of the fluorescence label intensity data mentioned with respect to Step S154. For example, the calculation process may include a process of acquiring fluorescence label intensity data by performing an unmixing process on the optical intensity data by using the data.

In addition, the automatic analysis processing section 11 may execute an Advanced analysis process such as a clustering process or a dimensional compression process.

The process of generating the analysis result data may be executed by using the analysis command mentioned with respect to Step S154. For example, the generation process may include generation of analysis result data including one or more of a plot image, a clustering result display view, and statistics on the basis of the fluorescence label intensity data by using the analysis command.

The automatic analysis processing section 11 (particularly, the virtual server) stores, for example, on the analysis result data storage section 16, the analysis result data generated in Step S155. In addition, the automatic analysis processing section 11 stores, for example, on the optical data storage section 15, the fluorescence label intensity data generated in Step S155. For example, these storage processes may be performed after the process in Step S155, or may be performed after the process in Step S156 or Step S157.

In Step S156, the automatic analysis processing section 11 (particularly, the virtual server) generates to-be-output data to be output on the data analysis client terminal 20. For example, the automatic analysis processing section 11 can identify or extract to-be-output data in or from the analysis result data generated in Step S155. The to-be-output data may be part of the analysis result data. For example, the to-be-output data includes one or more of a two-dimensional plot image, a spectral plot image, and a clustering result display view. The to-be-output data may include data for generating these images and, on the other hand, may not include the fluorescence label intensity data used in the analysis process. The to-be-output data may be configured to allow the data analysis client terminal 20 to edit these images. As a result, the data analysis client terminal 20 can execute a process of editing output data, and this data can easily be used in the interactive analysis process described later. In addition, the to-be-output data may include numerical data such as statistical analysis result data, for example.

In the present specification, "analysis result data" means data generated by an analysis process in the server system 10. "To-be-output data" is part of the analysis result data and is, in particular, data to be used for outputting analysis results on the data analysis client terminal 20.

In Step S157, the automatic analysis processing section 11 (particularly, the virtual server) ends the analysis process, and proceeds to a process in Step S105.

In Step S105, the automatic analysis processing section 11 (particularly, the virtual server) transmits an automatic analysis completion notification to the data analysis client terminal 20. The automatic analysis completion notification may be a mail or may be a notification by a notification method using a server-side Push technology. In response to the end of the transmission of the automatic analysis completion notification, the automatic analysis processing section 11 stops the virtual server. As a result, it is possible to avoid extra extension of the active time of the virtual server, and the cost of using the server is reduced.

In Step S106, the data analysis client terminal 20 receives the automatic analysis completion notification. The data analysis client terminal 20 causes the output apparatus to output the automatic analysis completion notification. In addition, in response to reception of the automatic analysis completion notification, the data analysis client terminal 20 may display a screen for inquiring of a user whether to transmit a to-be-output data request to the server system 10, for example. For example, a button for causing the data analysis client terminal 20 to transmit the to-be-output data request to the server system 10 may be arranged on the screen.

In Step S107, the data analysis client terminal 20 transmits the to-be-output data request to the server system 10. For example, in response to a click or selection of the button on the screen by the user of the data analysis client terminal 20, the data analysis client terminal 20 can execute the transmission.

In Step S108, the server system 10 receives the to-be-output data request.

In Step S109, the server system 10 transmits the to-be-output data generated in Step S104 to the data analysis client terminal 20.

In Step S110, the data analysis client terminal 20 receives the to-be-output data transmitted from the server system 10.

In Step S111, the data analysis client terminal 20 (particularly, the processing section 21) causes the output apparatus to output the to-be-output data. An example of the to-be-output data output on the output apparatus is depicted in FIG. 23. As depicted in FIG. 23, the output apparatus displays a window displaying the to-be-output data. Seven plot images are displayed on the lower left of the window. Further, one clustering result display view is displayed on the right of the window. Moreover, statistics data (the number of events, Parent%, and Total/) corresponding to each gate is displayed on the upper left of the window. In such a manner, the window can display image data and/or statistics data based on the to-be-output data. As described above, the image data may include one or more plot images and/or one or more clustering result display views. In addition, the statistics data may include one or more statistics.

In the automatic analysis process described above, the virtual server can be made active only when necessary. Accordingly, as compared with a server that is active at any time, the running cost can be reduced significantly. In addition, it is also possible to attain scalability.

(4-2) Example of Automatic Analysis Process

An example of an automatic analysis process flow in a case where the server system 10 is Amazon Web Services (trademark) is explained below with reference to FIGs. 14 and 15.

In Step S101, the data acquisition client terminal 30 acquires optical intensity data of a biosample from the biosample analyzing apparatus 40.

In Step S102, the data acquisition client terminal 30 transmits the optical intensity data to the server system 10. Preferably, the data acquisition client terminal 30 may execute the transmission in response to acquisition of the optical intensity data from the biosample analyzing apparatus 40 in Step S101.

In Step S103, the server system 10 receives, via the network 50, the optical intensity data transmitted from the data acquisition client terminal 30. The optical intensity data is uploaded to a bucket of Amazon S3 that functions as the optical data storage section 15.

In Step S104, the server system 10 executes an automatic analysis process on the optical intensity data. Details of the process in Step S104 are explained below with reference to FIG. 15.

In Step S151, for example, in response to uploading of an object including the optical intensity data to the bucket of Amazon S3 that functions as the optical data storage section 15, AWS Lambda that functions as the automatic analysis processing section 11 starts the automatic analysis process in Step S151.

In Step S152, the AWS Lambda reserves, on the server system 10, computation resources for executing an analysis process described later. Particularly, as a virtual server for executing an analysis process in Step S155 described later, an Amazon EC2 instance is created. In response to the creation of the Amazon EC2 instance, AWS Lambda may be ended.

In Step S153, the instance created in Step S152 downloads the object including the optical intensity data stored on the bucket.

In Step S154, the instance created in Step S152 acquires data necessary for the analysis, the data being stored in advance on Amazon Aurora. The data necessary for the analysis may be transmitted simultaneously with the optical intensity data in Step S102, and then stored in advance on Amazon Aurora.

In Step S155, for example, by using the spectral reference data, the instance calculates fluorescence label intensity data from the optical intensity data. The fluorescence label intensity data can be stored on the bucket.

In addition, the instance generates analysis result data by using the calculated fluorescence label intensity data.

The instance stores, on the bucket, the analysis result data generated in Step S155. In addition, the instance can also store, on the bucket, the fluorescence label intensity data generated in Step S155.

In Step S156, the instance generates to-be-output data from the analysis result data, for example. For example, the instance identifies or extracts the to-be-output data in or from the analysis result data.

In Step S157, the instance ends the analysis process. In response to the end of the analysis process, the instance proceeds to the process in Step S105.

Note that the number of pieces of the optical intensity data acquired in Step S101 may be one or greater than one. In a case where there are multiple pieces of the optical intensity data, for example, the multiple pieces of optical intensity data may be multiple pieces of optical intensity data acquired by performing measurement of each of multiple biosamples.

In this case, in Step S102, the data acquisition client terminal 30 transmits the multiple pieces of optical intensity data to the server system 10. In addition, the data acquisition client terminal 30 can also transmit the data necessary for the analysis to the server system 10.

Next, in Step S103, the server system 10 can record the multiple pieces of optical intensity data and/or the data necessary for the analysis on a database such as Amazon DynamoDB, for example.

Next, in Step S104, the instance can execute the automatic analysis process on each of the multiple pieces of optical intensity data. The instance may update the database upon completion of the automatic analysis process. The instance may proceed to the process in Step S105 in response to the end of the analysis process on all of the multiple pieces of optical intensity data.

In Step S105, the instance transmits an automatic analysis completion notification to the data analysis client terminal 20. In response to the end of the transmission of the automatic analysis completion notification, the instance may be stopped.

In Step S106, the data analysis client terminal 20 receives the automatic analysis completion notification. The data analysis client terminal 20 causes the output apparatus to output the automatic analysis completion notification. In addition, in response to reception of the automatic analysis completion notification, the data analysis client terminal 20 may display a screen for inquiring of a user whether to transmit a to-be-output data request to the server system 10, for example. For example, a button for causing the data analysis client terminal 20 to transmit the to-be-output data request to the server system 10 may be arranged on the screen.

In Step S107, the data analysis client terminal 20 transmits the to-be-output data request to the server system 10. For example, in response to a click or selection of the button on the screen by the user of the data analysis client terminal 20, the data analysis client terminal 20 can execute the transmission.

In Step S108, the server system 10 receives the to-be-output data request.

In Step S109, the server system 10 transmits the to-be-output data generated in Step S104 to the data analysis client terminal 20.

In Step S110, the data analysis client terminal 20 receives the to-be-output data transmitted from the server system 10.

In Step S111, the data analysis client terminal 20 causes the output apparatus to output the to-be-output data.

(4-3) To-Be-Output Data Acquisition Process

The to-be-output data acquisition process by the information processing system 1 is explained below with reference to FIG. 16.

In Step S201, the data analysis client terminal 20 transmits a to-be-output data request to the server system 10. The request may include information to be used by the server system 10 to acquire or generate to-be-output data. For example, the request includes information to be used for identifying analysis result data and/or information to be used for generating to-be-output data from analysis result data. The information transmitted in Step S201 may include user authentication information regarding the data analysis client terminal 20, in addition to the request. The transmission in Step S201 may be performed via the network 50.

In Step S202, the server system 10 receives the to-be-output data request transmitted from the data analysis client terminal 20 via the network 50.

In Step S203, the server system 10 (particularly, the to-be-output data generating section 13) may acquire to-be-output data from the analysis result data storage section 16 or may generate to-be-output data from analysis result data stored on the analysis result data storage section 16. The to-be-output data may be part of the analysis result data. For example, the to-be-output data includes one or more of a two-dimensional plot image, a spectral plot image, and a clustering result display view. The to-be-output data may include data for generating these images and, on the other hand, may not include the fluorescence label intensity data used in the analysis process. The to-be-output data may be configured to allow editing of these images. As a result, for example, the data analysis client terminal can execute a process of editing output data. In addition, the to-be-output data may include numerical data such as statistical analysis result data.

Processes by the to-be-output data generating section 13 may be executed by a virtual server that is active at any time in the server system 10. Since the virtual server is active at any time, the to-be-output data acquisition process can be executed at a high speed.

In addition, the to-be-output data generating section 13 may be configured as a serverless architecture.

For example, in a case where the server system 10 is Amazon Web Services (trademark), the to-be-output data acquisition or generation process by the to-be-output data generating section 13 may be executed in a container generated or managed by Amazon ECS, for example. For example, AWS Fargate can execute the process in the container. For example, AWS Fargate can acquire to-be-output data stored in Amazon S3 or generate to-be-output data from analysis result data stored in Amazon S3.

In Step S204, the server system 10 transmits the to-be-output data to the data analysis client terminal 20 via the network 50.

In Step S205, the data analysis client terminal 20 receives the to-be-output data via the network 50.

In Step S206, the data analysis client terminal 20 causes the output apparatus to output the to-be-output data.

(4-4) Interactive Analysis Process

The flow of an interactive analysis process by the information processing system 1 is explained below with reference to FIG. 17. The analysis process may be executed subsequent to the automatic analysis process explained in (4-1) above or may be executed subsequent to the to-be-output data acquisition process explained in

(4-3) above.

In Step S301, the data analysis client terminal 20 transmits an interactive analysis process start request to the server system 10 via the network 50. Prior to the transmission, the data analysis client terminal 20 can cause the output apparatus to output to-be-output data. For example, Step S301 may be executed next to Step S107 explained in (4-1) above or may be executed next to Step S206 explained in (4-3) above.

For example, in a case where the to-be-output data acquisition process explained in (4-3) above is performed, the data analysis client terminal 20 causes the output apparatus to output a window like the one depicted in FIG. 20. Then, in Step S206, as depicted in FIG. 21, acquired to-be-output data (plot data) is displayed on the window.

For example, the request includes data for identification to be used for identifying analysis-subject data which is the subject of the interactive analysis process. For example, the data for identification includes information identifying a biosample and information identifying an analysis performed on the biosample.

In Step S302, the server system 10 receives the request via the network 50.

In Step S303, in response to reception of the request, the server system 10 (particularly, the interactive analysis processing section 12) reserves, on the server system 10, computation resources for executing the interactive analysis process. The computation resources function as a virtual server that executes the interactive analysis process.

In Step S304, the interactive analysis processing section 12 (particularly, the virtual server) acquires the analysis-subject data. With reference to the data for identification, the interactive analysis processing section 12 may identify the analysis-subject data that is to be acquired.

The analysis-subject data may include analysis result data from which the to-be-output data has been derived. In addition, the analysis-subject data may include optical intensity data and/or fluorescence label intensity data from which the analysis result data has been derived.

After the acquisition of the analysis-subject data in Step S304, the interactive analysis processing section 12 may include monitoring whether an analysis command described later has been received, that is, waiting until reception of the analysis command.

After the execution of Step S304 but before Step S305, a session for executing RPC (Remote Procedure Call) may be established between the server system 10 and the data analysis client terminal 20. For example, for the establishment, the server system 10 can transmit, to the data analysis client terminal 20, a notification for informing that computation resources have been reserved, for example. The establishment can be performed in response to reception of the notification. For example, the notification may be a notification by a notification method using a server-side Push technology. Alternatively, the establishment may be performed by state acquisition by polling from the data analysis client terminal 20 to the server system 10.

By the establishment of RPC, in and after Step S305, the server system 10 can execute the analysis process according to the analysis command transmitted from the data analysis client terminal 20. Both the server system 10 and the data analysis client terminal 20 may include a connecting section which is a functional element for executing RPC. The combination of the connecting section 14 of the server system 10 and the connecting section 23 of the data analysis client terminal 20 may execute RPC. Examples of these connecting sections include MagicOnion, but are not limited to this.

In Step S304, the interactive analysis processing section 12 (particularly, the virtual server) may acquire data necessary for an analysis. For example, the data necessary for the analysis can include data to be used for calculation of fluorescence label intensity data and data to be used in the fluorescence intensity labeled data analysis process.

The data to be used for calculation of fluorescence label intensity data can include spectral reference data, for example. The data is used for an unmixing process.

The data to be used in the fluorescence intensity labeled data analysis process can further include the analysis command.

In Step S305, the data analysis client terminal 20 accepts input of an analysis command for the to-be-output data. In Step S305, the data analysis client terminal 20 causes the output apparatus to display a window for accepting input of the analysis command. The user inputs the analysis command via the window. For example, the input analysis command may be an analysis command in which one or more of various types of settings commands included in the analysis command to be used for acquiring the to-be-output data output in Step S301 are changed. For example, the input analysis command includes at least one of a changed gate settings command, a changed plot settings command, a changed axis settings command, a changed axis display settings command, a changed statistics settings command, and a changed region settings command. In addition, the analysis command may include a changed clustering command.

In Step S306, the data analysis client terminal 20 transmits, to the server system 10, the analysis command input in Step S305.

In Step S307, the server system 10 receives the analysis command transmitted from the data analysis client terminal 20. In response to reception of the analysis command, processes in Steps S308 to S310 are executed. These processes may be event-driven analysis processes triggered by reception of the analysis command. That is, an interactive analysis process according to the present technology may be an event-driven analysis process.

For example, the process by the interactive analysis processing section 12 may be executed in a serverless manner, and the interactive analysis processing section 12 may be configured as what is generally called a serverless architecture. For example, in a case where the server system 10 is Amazon Web Services (trademark), the process by the interactive analysis processing section 12 may be executed by AWS Lambda, for example, and is particularly executed by a system including the combination of AWS Lambda and Amazon EC2.

In Step S308, the interactive analysis processing section 12 (particularly, the virtual server) executes a process of calculating fluorescence label intensity data from the optical intensity data. Then, the interactive analysis processing section 12 executes a process of generating analysis result data by using the calculated fluorescence label intensity data.

Note that, in a case where the fluorescence label intensity data is not changed, the interactive analysis processing section 12 may omit the process of calculating the fluorescence label intensity data, that is, may execute only the process of generating the analysis result data by using the existing fluorescence label intensity data.

The interactive analysis processing section 12 stores the analysis result data generated in Step S308 on the analysis result data storage section 16, for example. In addition, the interactive analysis processing section 12 stores the fluorescence label intensity data generated in Step S308 on the optical data storage section, for example. For example, these storage processes may be performed after the process in Step S308 or may be performed after the process in Step S310.

In Step S309, the interactive analysis processing section 12 generates to-be-output data to be output on the data analysis client terminal. For example, the interactive analysis processing section 12 can identify or extract to-be-output data in or from the analysis result data generated in Step S309. The to-be-output data may be part of the analysis result data. For example, the to-be-output data includes one or more of a two-dimensional plot image, a spectral plot image, and a clustering result display view. The to-be-output data may include data for generating these images and, on the other hand, may not include the fluorescence label intensity data used in the analysis process. The to-be-output data may be configured to allow editing of these images. As a result, for example, the data analysis client terminal can execute a process of editing output data. In addition, the to-be-output data may include numerical data such as statistical analysis result data.

In Step S309, the server system 10 transmits the generated to-be-output data to the data analysis client terminal 20. The transmission of the to-be-output data may be performed for each data unit included in the to-be-output data, or the whole to-be-output data may be transmitted collectively.

Regarding the former, for example, in one possible case, the to-be-output data includes two of statistics data and image data (one or more of a two-dimensional plot image, a spectral plot image, and a clustering result display view). For example, when to-be-output statistics data is generated, the server system 10 can transmit the statistics data, and thereafter, when to-be-output image data is generated, the server system 10 can transmit the image data. Alternatively, on the contrary, the image data may be transmitted first, and the statistics data may be transmitted next. In such a manner, the server system 10 can transmit to-be-output data in each data unit.

In Step S310, the data analysis client terminal 20 receives the to-be-output data.

In Step S311, the data analysis client terminal 20 causes the output apparatus to output the to-be-output data.

For example, as depicted in FIG. 22, a plot image which is obtained by partially changing the plot image in FIG. 21 is displayed on the window.

Processes in Steps S305 to S312 may be repeated on the to-be-output data output in Step S312. Every time these processes are repeated, the to-be-output data to be output on the output apparatus may be updated, and rendered again.

As described above, the analysis command input on the data analysis client terminal 20 and the interactive analysis process by the server system 10 are repeated, and in such a manner, the interactive analysis process by the server system 10 and the data analysis client terminal 20 is repeated.

After the end of the interactive analysis process, the interactive analysis processing section 12 stops the virtual server. As a result, it is possible to avoid extra extension of the active time of the virtual server, and the cost of using the server is reduced.

For example, the interactive analysis process may be ended when the data analysis client terminal 20 accepts an instruction to end the interactive analysis process. In response to acceptance of the end instruction, the data analysis client terminal 20 transmits an end instruction to the server system 10. In response to reception of the end instruction, the interactive analysis processing section 12 stops the virtual server. As a result, it is possible to avoid extra extension of the active time of the virtual server, and the cost of using the server is reduced.

Alternatively, the interactive analysis processing section 12 may stop the virtual server in response to an elapse of a predetermined length of time without reception of an analysis command from the data analysis client terminal 20 by the server system 10.

In the interactive analysis process described above, the virtual server can be made active only when necessary. Accordingly, as compared with a server that is active at any time, the running cost can be reduced significantly. In addition, it is also possible to attain scalability.

(4-5) Example of Interactive Analysis Process

An example of the interactive analysis process flow in FIG. 17 in a case where the server system 10 is Amazon Web Services (trademark) is further explained below with reference to FIGs. 18 and 19.

As depicted in FIG. 18, in Step S301, the data analysis client terminal 20 transmits an interactive analysis process start request to the server system 10 via the network 50.

In Step S302, the server system 10 receives the request via the network 50.

In Step S303, in response to reception of the request, AWS Lambda that functions as the interactive analysis processing section 12 reserves, on the server system 10, computation resources for executing the interactive analysis process. Particularly, AWS Lambda causes Amazon ECS to execute a container (particularly, a Docker container) in Amazon EC2. In the container, a process of a virtual server that executes an analysis process described later is executed. In addition, the process of the virtual server may be executed without constructing the container.

In Step S304, the instance acquires analysis-subject data stored on Amazon S3, for example.

In addition, in Step S304, the instance can acquire data necessary for the analysis. For example, the instance acquires data necessary for the analysis, the data being stored in advance on Amazon Aurora (e.g., data to be used for calculation of fluorescence label intensity data, etc.).

After the execution of Step S304 but before Step S305, a session for executing RPC (Remote Procedure Call) may be established between the server system 10 and the data analysis client terminal 20. For example, the server system 10 can transmit, to the data analysis client terminal 20, a notification for informing that computation resources have been reserved in Amazon EC2. The establishment may be performed in response to reception of the notification. Both the server system 10 and the data analysis client terminal 20 may include MagicOnion as a connecting section which is a functional element for executing RPC.

After the acquisition of the analysis-subject data in Step S304, the instance monitors whether an analysis command described later has been received, that is, waits until reception of the analysis command.

As depicted in FIG. 19, in Step S305, the data analysis client terminal 20 accepts input of an analysis command for the to-be-output data.

In Step S306, the data analysis client terminal 20 transmits, to the server system 10, the analysis command input in Step S305.

In Step S307, the server system 10 receives the analysis command transmitted from the data analysis client terminal 20. In response to reception of the analysis command, processes in Steps S308 and S309 are executed. These processes may be event-driven analysis processes triggered by reception of the analysis command. That is, an interactive analysis process according to the present technology may be an event-driven analysis process.

In Step S308, the instance executes a process of calculating fluorescence label intensity data from the optical intensity data. Then, the instance executes a process of generating analysis result data by using the calculated fluorescence label intensity data.

Note that, in a case where the fluorescence label intensity data is not changed, the instance may omit the fluorescence label intensity data, that is, may execute only the process of generating the analysis result data by using the existing fluorescence label intensity data.

The instance stores, for example, on an S3 bucket, the analysis result data generated in Step S308. In addition, the interactive analysis processing section 12 also stores, on the bucket, the fluorescence label intensity data generated in Step S308.

In Step S309, the instance generates to-be-output data from the analysis result data, and then transmits the generated to-be-output data to the data analysis client terminal 20.

In Step S310, the data analysis client terminal 20 receives the to-be-output data.

In Step S311, the data analysis client terminal 20 causes the output apparatus to output the to-be-output data.

Processes in Steps S305 to S311 may be repeated on the to-be-output data output in Step S311. That is, the analysis command input on the data analysis client terminal 20 and the interactive analysis process by the server system 10 are repeated, and in such a manner, the interactive analysis process by the server system 10 and the data analysis client terminal 20 is repeated.

(5) Settings of Region

The server system 10 may include a group of servers each of which is present in one of multiple geographically dispersed data centers. The multiple data centers may be present dispersedly in multiple countries, for example. Each data center can be called a region.

In the present disclosure, depending on a position where the data analysis client terminal 20 and/or the data acquisition client terminal 30 are/is present, the server system 10 can identify a data center that is included in the multiple data centers and that is to execute an automatic analysis process and/or an interactive analysis process according to the present disclosure, and a server in the identified data center can execute the processes. Preferably, from the multiple data centers, the server system 10 can identify a data center that is closer to the position, as a data center to execute the processes.

In the present disclosure, according to preset information such as information related to the position where the data analysis client terminal 20 and/or the data acquisition client terminal 30 are/is present or contract information, the server system 10 can identify a data center that is included in the multiple data centers and to or on which data to be used in processes according to the present disclosure is uploaded or stored, and the data can be uploaded to or stored on a server in the identified data center. Preferably, from the multiple data centers, the server system 10 can identify a data center that is closer to the position, as a data center to or on which the data is to be uploaded or stored.

Particularly preferably, a server to execute the processes and a server to or on which the data is to be uploaded or stored may be present in the same data center.

By selecting a data center as described above, it is possible to increase the speed of processes according to the present disclosure. For example, by adopting a configuration that makes it possible to set, for each user, contract, or the like, a data center on which data is to be stored, a data center geographically close to a user can be used, and this makes it possible to increase the speed of data uploading, increase the speed of responses at a time of an interactive analysis, and so on.

(6) Data Migration

In the present disclosure, the optical data storage section may include two or more types of storages with different access speeds. Further, in a case where a predetermined condition is satisfied, the server system may execute a migration process of migrating the optical intensity data and/or the fluorescence label intensity data stored on a storage with a higher access speed (hereinafter, also referred to as a "hot storage") to a storage with a lower access speed (hereinafter, also referred to as a "cold storage").

Typically, storage services provided by a cloud platform often include several types of storage services like what are generally called hot storages that allow swift access to data but instead require higher unit prices and what are generally called cold storages that, conversely, have lower response speeds for data access but instead require lower unit prices. In the present disclosure, by executing the migration process as described above, it becomes possible to migrate data from a hot storage to a cold storage, and it becomes possible to reduce the costs. The predetermined condition for executing the migration process may be preset by a user. For example, the predetermined condition may be a condition that the migration process is executed in a case where a predetermined number of days have passed without data access after the latest access to the data or other conditions.

Preferably, the server system executes the migration process after performing a compression process on subject data of the migration process. As a result, the storage costs can be reduced further.

In order to access data stored on a cold storage, the server system 10 may perform data copy from the cold storage to a hot storage. In addition, in order to access compressed data, the server system 10 can also execute a decompression process at a time of the data copy.

The server system 10 may include a database including information regarding storages where data is present (which cold storage or which hot storage has the data stored thereon). The server system 10 or the data analysis client terminal 20 can access data in a cold storage simply and conveniently by referring to the database.

(7) Use of External Storages or Computation Resources

As described in (4-1) above, the automatic analysis processing section included in the information processing system according to the present disclosure may start an automatic analysis process in response to storage of optical intensity data on the optical data storage section 15. Whereas the optical data storage section 15 is present in the server system 10 in the embodiment explained in (4-1) above, the optical data storage section 15 may be present outside the server system 10 in the present disclosure. For example, an external storage present outside the server system 10 may be used as the optical data storage section 15 on which optical intensity data is stored.

The external storage present outside the server system 10 may be an online storage owned by a user of the information processing system according to the present disclosure, for example. In response to storage of optical intensity data on the external storage, the server system 10 (particularly, the automatic analysis processing section 11) may start the automatic analysis process described in (4-1) above. For example, the external storage can notify the server system 10 that the optical intensity data has been stored. By being triggered by reception of the notification, the automatic analysis processing section 11 can execute the event-driven analysis process described in (4-1) above.

In this case, Steps S152 and S154 to S157 may be executed as described in (4-1) above. In Step S153, the automatic analysis processing section 11 downloads the optical intensity data stored on the external storage used as the optical data storage section 15.

In addition, whereas the automatic analysis processing section 11 reserves, on the server system 10, computation resources for executing the analysis process in the explanation in (4-1) above, the computation resources may be computation resources outside the server system 10. For example, computation resources for executing the analysis process may be reserved in an information processing apparatus that is present outside the server system 10.

In recent years, research institutes such as universities or corporate laboratories have an account of an online storage or a cloud platform, for example, store data on the cloud, and perform an analysis by using computation resources on the cloud. By the information processing system according to the present disclosure using an external online storage as a data storage destination or executing an analysis process by using external computation resources as described above, users can further reduce the operating costs of server systems.

(8) Division of Analysis-Subject Data

In Step S155 explained in (4-2) above and in Step S309 explained in (4-4) above, a process of calculating fluorescence label intensity data from optical intensity data is executed. For example, the process includes a fluorescence correction process or an unmixing process, and these processes are executed, particularly, in a case where the biosample analyzing apparatus is a bioparticle analyzing apparatus such as a flow cytometer. Data acquired by the bioparticle analyzing apparatus can be divided in units of events (i.e., in units of measurement results of each bioparticle).

In view of this, in a calculation process of calculating fluorescence label intensity data from optical intensity data, the information processing system (particularly, the server system) according to the present disclosure may first divide the optical intensity data in units of events, and then execute the calculation process for each piece of the optical intensity data obtained by the division. The calculation process for respective pieces of the optical intensity data obtained by the division may be executed in parallel and simultaneously, for example. For example, the server system (particularly, the automatic analysis processing section) creates multiple AWS Lambdas, and then allocates a piece of optical intensity data obtained the by the division to each virtual server. Then, each virtual server executes the calculation process on the allocated piece of the optical intensity data obtained by the division.

By executing the division process as described above, it is possible to increase the processing speed. Further, it is also possible to make the process more efficient.

(9) Data Sharing

The metadata (particularly, the analysis settings data), the optical intensity data, and the fluorescence label intensity data described in (4-1) above can be stored on a storage or a storage section included in the server system 10 in the present disclosure. In the present disclosure, these pieces of data may be stored in the server system 10 in a state where they are associated with each other. Since these pieces of data are associated with each other, reproduction of measurement and/or reproduction of an analysis of measurement results become(s) easier.

In addition, the additional data, the data to be used for calculation of the fluorescence label intensity data, and the data to be used in the fluorescence intensity labeled data analysis process described in (4-1) above may also be stored in the server system 10 in a state where they are associated with each other. Since these pieces of data are associated with each other, reproduction of measurement and/or reproduction of an analysis of measurement results become(s) easier.

In addition, these pieces of data (particularly, these pieces of data that are associated with each other) may be available only on one data analysis client terminal and/or data acquisition client terminal, or may be available on two or more data analysis client terminals and/or data acquisition client terminals. That is, these pieces of data can be shared by the two or more data analysis client terminals and/or data analysis client terminals.

For example, in the present disclosure, multiple data analysis client terminals can share one or more of optical intensity data, fluorescence label intensity data, and analysis settings data in the server system. Particularly preferably, multiple data analysis client terminals can share analysis settings data in the server system.

In addition, in the present disclosure, multiple data acquisition client terminals can share one or more of optical intensity data, fluorescence label intensity data, and analysis settings data in the server system. Particularly preferably, multiple data acquisition client terminals can share analysis settings data in the server system.

Since the information processing system (or the server system, the data analysis client terminal, or the data acquisition client terminal included in the system) is configured in the above-described manner in the present disclosure, information regarding measurement and/or an analysis by a certain user can be reused by another user, and reproduction of measurement and/or reproduction of an analysis of measurement results become easier.

(10) Standardization of To-Be-Output Data

The to-be-output data generated by the automatic analysis process described in (4-1) above, the to-be-output data acquired or generated by the to-be-output data acquisition process described in (4-3) above, and the to-be-output data generated by the interactive analysis process described in (4-4) above may each be configured such that they can be output on windows (particularly, worksheets) having the same user interface. For example, these three pieces of to-be-output data may have the same metadata to be used for being output by an output apparatus. As a result, for example, it becomes easier to use, in the interactive analysis process, the to-be-output data generated by the automatic analysis process or the to-be-output data acquired or generated by the to-be-output data acquisition process. In addition, the analysis command input in Step S305 of the interactive analysis process can also be used for executing the automatic analysis process on newly acquired optical intensity data.

Since the to-be-output data is displayed on worksheets having similar user interfaces in such a manner, a user can view and execute the automatic analysis process, the to-be-output data generation process, and the interactive analysis process on the consistent user interfaces. In addition, since various types of analysis settings data used in measurement are stored along with analysis results, measurement and analyses can easily be reproduced with use of similar settings. By combining these mechanisms and Standardization functionalities for data between apparatuses, it becomes possible to easily realize reproduction of identical experiments on multiple different apparatuses.

(11) Example 1 of Output Control of Clustering Result Display View

As described in (4-1) above, in the present disclosure, the data analysis client terminal 20 causes the output apparatus to output the to-be-output data including the clustering result display view. For example, the clustering result display view may be a clustering result display view like the one depicted in FIG. 23. The clustering result display view depicted in FIG. 23 is an output result obtained when a cluster algorithm called FlowSOM is executed, and is also called a star chart.

In a case where the number of markers to be used in an analysis is great, the number of markers included in the to-be-output data also increases. If data related to all the markers is displayed on the clustering result display view in such a case, the contents of the clustering result display view becomes complicated, and it becomes difficult to grasp the expression level of each marker. For example, if the number of the types of markers displayed on each cluster increases on a star chart or a population pie chart in FlowSOM, it becomes difficult to grasp the expression level of each cluster in some cases.

In a preferred embodiment according to the present disclosure, the data analysis client terminal 20 may be configured to make it possible to change the number of markers to be displayed on a clustering result display view, and, more specifically, may be configured to make it possible to select markers to be included in each cluster in the clustering result display view.

Since it is made possible to select markers to be included in each cluster, the clustering result display view can be adjusted according to the purpose of a user. Further, since the number of markers to be included in the clustering result display view can be adjusted, it becomes easier to grasp the expression levels of markers.

For example, in response to selection of one or more markers from all the markers included in the to-be-output data, the data analysis client terminal 20 may form a clustering result display view related to the one or more selected markers. The formed clustering result display view may be one that does not include data related to one or more unselected surface markers.

That is, in response to the selection, the data analysis client terminal 20 can form a clustering result display view on the basis of data regarding the one or more selected markers. The clustering result display view may be a star chart or a pie chart, but is not limited to these.

An example of a case where the clustering result display view is a star chart is further explained below with reference to the drawings.

Supposed is a case where, in Step Sill explained in (4-1) above, on the basis of to-be-output data, the data analysis client terminal 20 causes a screen of the output apparatus to display a star chart 500 like the one depicted in FIG. 24, for example, as a clustering result display view. Further, it is supposed that a user desires to reduce the number of markers to be displayed on the star chart.

In this case, for example, in response to selection (a click or a touch) of the star chart by the user or selection of a predetermined button by the user, the data analysis client terminal 20 causes the output apparatus to display a marker-selection window 501 like the one depicted in FIG. 25. The window includes a color coding element 502 representing a marker group displayed in the star chart and a color corresponding to each marker and a marker list 503 of markers displayed in the star chart.

Here, the marker list is configured to allow selection of markers to be displayed in the star chart.

In addition, whereas the color coding element is depicted like pie display in FIG. 25, it is sufficient if the color coding element represents associations between markers and colors, or another display format may be adopted.

Next, from the marker list, the user selects markers that she/he desires to display in the star chart. For example, a marker list 512 depicted in FIG. 26 represents a state where the user has selected five markers (shaded in gray).

In response to selection of the five markers, the data analysis client terminal 20 causes a color coding element 513 to display only the colors of the selected markers as represented by the color coding element in FIG. 26. As a result, the user can grasp the display contents of the star chart after the marker selection.

In response to selection of the five markers, the data analysis client terminal 20 also changes the star chart depicted in FIG. 24. For example, in response to selection of the five markers, the data analysis client terminal 20 changes the star chart 500 into a star chart 510 depicted in FIG. 27.

As can be known from a comparison between a left enlarged view 504 in FIG. 24 and a left enlarged view 514 in FIG. 27, the data analysis client terminal 20 changes data elements (the number of colors) displayed in each cluster.

As described above, the data analysis client terminal 20 may be configured to change a clustering result display view in response to selection of markers in the marker list. Then, as described above, the clustering result display view may be changed such that only data (data based on expression levels, colors corresponding to markers, and/or the like) of markers selected on the marker list is displayed.

Note that, as described above, the data analysis client terminal 20 may change the displayed clustering result display view in conjunction with a marker selection operation on the marker list 512.

Alternatively, the data analysis client terminal 20 may change the displayed star chart, or change the displayed clustering result display view, in response to selection of a predetermined button (e.g., a predetermined button on the marker-selection window; for example, a Close button depicted in FIG. 26, etc.).

Whereas the number of markers is reduced in the example depicted in the description above, the number of markers may be increased. Further, in conjunction with selection of markers, the data analysis client terminal 20 may change the color coding element on the marker-selection window. In addition, in conjunction with selection of markers, the data analysis client terminal 20 may also change the clustering result display view.

For example, FIG. 28 depicts a state where ten markers are selected in a marker list 522 on a marker-selection window 521. In response to the selection, the data analysis client terminal 20 causes a color coding element 523 color-coded in ten colors to be displayed. Then, in response to the selection, the data analysis client terminal 20 may display a clustering result display view 520 like the one depicted in FIG. 29.

In addition, FIG. 30 depicts a state where three markers are selected in a marker list 532 on a marker-selection window 531. In response to the selection, the data analysis client terminal 20 causes a color coding element 533 color-coded in three colors to be displayed. Then, in response to the selection, the data analysis client terminal 20 may display a clustering result display view 530 like the one depicted in FIG. 31.

In addition, it may also be made possible to change the order of display of markers in a star chart as desired. That is, the data analysis client terminal 20 may be configured to allow a user to change the order of display of markers in a star chart. For example, the order of display may be changed in response to a user operation on the marker list or the color coding element on the marker-selection window, or may be executed in response to a user operation on the star chart itself.

For example, in response to a predetermined operation (e.g., a drag operation) by a user to change the positional relation of two or more selected markers in the marker list, the data analysis client terminal 20 can change the order of display.

Alternatively, in response to a predetermined operation (e.g., a drag operation) by a user to change the positional relation of two or more pies in the color coding element, the data analysis client terminal 20 can change the order of display. By a similar operation on nodes in the star chart, the data analysis client terminal 20 may change the order of display.

In addition, whereas the data analysis client terminal 20 changes the clustering result display view in the explanation above, the server system 10 may change the clustering result display view.

For example, in response to selection of markers on the marker-selection window 501 by a user, the data analysis client terminal 20 transmits data related to the selected markers to the server system 10. Then, on the basis of the data related to the selected markers, the server system 10 generates a clustering result display view that has been changed, and then transmits, to the data analysis client terminal 20, the changed clustering result display view. Then, the data analysis client terminal 20 may cause the output apparatus to display the changed clustering result display view.

(12) Example 2 of Output Control of Clustering Result Display View

There are often a large number of clusters (e.g., nodes in FlowSOM, etc.) in the clustering result display view explained in (11) above. For example, as the number of types of cells which are the subjects of an analysis increases, the number of clusters also increases. There is a functionality of grouping clusters having similar marker expression tendencies which are called metaclusters in FlowSOM, but a star chart or a population pie chart displays clusters in units of clusters. If all the clusters are displayed in the clustering result display view in such a case, it becomes difficult to grasp, from the clustering result display view, the tendency or overview (e.g., the tendency of expression levels in units of metaclusters, etc.) of marker expression levels of a sample as a whole on which measurement has been performed, in some cases.

FIG. 32 depicts an example of a clustering result display view (star chart) including a large number of nodes. Since a clustering result display view 600 in FIG. 32 displays a large number of clusters, for example, a large number of clusters displayed in a region 601 surrounded by dotted lines overlap each other, and the tendency of expression levels in these clusters is difficult to check. The tendency of the expression levels in this region is difficult to check even if the region is expanded as depicted on the right in FIG. 32, for example. In addition, it is more difficult to grasp the tendency or features of expression levels in units of metaclusters formed by grouping multiple clusters. As a result, it is difficult to grasp an overview of the expression tendency of the sample as a whole.

In a preferred embodiment according to the present disclosure, the data analysis client terminal 20 may be configured to be able to output a star chart or a population pie chart in units of metaclusters each formed by grouping one or more clusters having similar expression tendencies.

The metaclusters may be formed by automatically grouping clusters having similar marker expression tendencies by an algorithm, or, alternatively, the metaclusters may be formed in response to selection of one or more clusters by a user her/himself.

Such a clustering result output view in units of metaclusters, that is, a metacluster chart, makes it easier to grasp the tendency of expression levels.

An example of a metacluster chart formed from a clustering result display view is depicted in FIG. 33. On the left in FIG. 33, the clustering result display view 600 explained above is displayed. For example, in response to acceptance of a predetermined operation by a user, the data analysis client terminal 20 generates a metacluster chart 602 like the one depicted on the right in FIG. 33 from the clustering result display view 600. Here, the predetermined operation may be an operation such as a click or a touch of a predetermined operation button, for example.

Multiple clusters in the clustering result display view 600 can be classified into seven cluster groups (called a metacluster 611 to a metacluster 617) as depicted in FIG. 33. Each metacluster has a common feature (e.g., a common feature related to the expression situations of markers). Cluster groups belonging to the same metacluster are coded with the same color. In addition, different metaclusters are coded with mutually different colors.

For each of these seven metaclusters, the data analysis client terminal 20 generates a metacluster node on the basis of data regarding one or more clusters belonging to the metacluster. The metacluster chart 602 including the seven generated metacluster nodes 621 to 627 are depicted on the right in FIG. 33.

The color of the metacluster node 621 is the same as the color of the metacluster 611. Similarly, the colors of the metacluster nodes 622 to 627 are the same as the colors of the metaclusters 612 to 617, respectively. Since a metacluster and a metacluster node are coded with the same color before and after the formation of the metacluster chart as described above, it becomes easier to understand the relation between the metacluster and the metacluster node.

The data analysis client terminal 20 may set the size (the size of the diameter of a circle in FIG. 33) of a metacluster node according to quantification data such as the number of events or the number of cells belonging to the metacluster node, for example. In FIG. 33, the larger the number of events included in a metacluster node is, the larger the diameter of the corresponding circle is.

In addition, in the metacluster chart, the data analysis client terminal 20 may arrange each metacluster node at a position identified on the basis of the position(s) of one or more clusters belonging to the metacluster node. For example, the data analysis client terminal 20 may identify the position of each metacluster node in a metacluster chart on the basis of the number of events included in each of one or more clusters belonging to the metacluster node and the position(s) of the one or more clusters. In one example, the position of each metacluster node may be the centroid position of the position(s) of one or more clusters integrated into the metacluster node.

Note that identification of the positions of metacluster nodes like the one described above may not be performed in a metacluster chart. For example, as depicted in a metacluster chart 603 in FIG. 34, the data analysis client terminal 20 may arrange one or more generated metacluster nodes such that predetermined numbers of lines and/or columns are formed, or may arrange one or more generated metacluster nodes in a grid.

In addition, whereas the data analysis client terminal 20 forms a metacluster chart from a clustering result display view in the explanation above, the formation of the metacluster chart may be executed by the server system 10.

For example, a user performs a predetermined operation for forming a metacluster chart in order to form the metacluster chart from a certain clustering result display view. In response to acceptance of the predetermined operation, the data analysis client terminal 20 transmits, to the server system 10, instruction data for generating a metacluster chart from the clustering result display view. In response to reception of the instruction data, the server system 10 generates a metacluster chart from the clustering result display view, and then transmits the metacluster chart to the data analysis client terminal 20. Then, the data analysis client terminal 20 may cause the output apparatus to display the metacluster chart.

(13) Association between Metaclusters in Clustering Result Display View and Two-Dimensional Plot

As described in (12) above, multiple clusters in a clustering result display view can be classified into metaclusters each including one or more clusters having a common feature. Here, if the metaclusters in the clustering result display view can be associated with events in a two-dimensional plot, it is possible to intuitively grasp events belonging to metaclusters.

In a preferred embodiment according to the present disclosure, in response to selection of a metacluster in a clustering result display view, the data analysis client terminal 20 may display a two-dimensional plot such that events belonging to the metacluster can be identified from events included in the two-dimensional plot. For example, the data analysis client terminal 20 may give a color given in common to a metacluster to events belonging to the metacluster in a two-dimensional plot.

By such display of the two-dimensional plot, it is possible to intuitively grasp events belonging to a metacluster, and further, backgating becomes also possible.

A display control technique for a two-dimensional plot described above is further explained below.

Supposed is a case where, in Step S111 explained in (4-1) above, as depicted in FIG. 35, the data analysis client terminal 20 causes the screen of the output apparatus to display the clustering result display view 600 as well as two-dimensional plots 630 and 631 on the basis of to-be-output data. The clustering result display view 600 is the one explained in (12) above, and multiple clusters in the clustering result display view can be classified into seven metaclusters as depicted in FIG. 36.

As depicted in FIG. 37, when a user performs a drag operation with a mouse cursor from the position of a reference sign 632 to the position of a reference sign 633, a region 634 surrounded by broken lines is selected. When the region 634 is selected, metaclusters 611 to 617 overlapping the region are also selected. In response to selection of the metaclusters 611 to 617, as depicted in FIG. 37, the data analysis client terminal 20 gives colors given to the metaclusters to events belonging to the metaclusters in the two-dimensional plots 630 and 631. By the display technique of giving colors in such a manner, it is possible to intuitively grasp events belonging to a metacluster, and further, backgating also becomes possible.

Note that, whereas multiple metacluster are selected by a drag operation in the example depicted in FIG. 37, for example, one or more metaclusters may be selected by a clicking operation. Also in this case, events belonging to the selected metaclusters may be coded with colors given to the metaclusters.

(14) Control of Pie Display Axis in Star Chart

As depicted also in the drawings explained in (11) to (13) above, data regarding expression levels of a marker group is depicted by pie display in each node (cluster) in a star chart in some cases. For example, the length in a radial direction of a region (pie) displayed corresponding to a marker increases as the expression level of the marker increases. For example, the expression levels of multiple markers are depicted by pie display in a node 700 depicted in FIG. 38. The expression level of each marker corresponds to the length in a radial direction, and, for example, the expression level of a marker corresponding to a region denoted by a reference sign 701 corresponds to a length 702 in the radial direction. Note that, whereas the length 702 is represented by an arrow in FIG. 38, such an arrow may not be displayed in a star chart.

In the present disclosure, the data analysis client terminal 20 may be configured to be able to change axis settings of pie display in each node. For example, the data analysis client terminal 20 may change the axis settings of pie display such that the axis scale of pie display in a certain node is made correspond to the axis scale of a two-dimensional plot corresponding to the node. Here, each node which is a group of events may not be a cluster formed by executing clustering. For example, each node may be formed on the basis of an event group present in a gate, in a case where the gate is created on a two-dimensional plot.

Since the axis settings can be changed as described above, it becomes easier to grasp the degrees of expression levels. Moreover, it becomes also possible to grasp the relation with a two-dimensional plot more precisely.

Examples of the axis scale of a two-dimensional plot include Linear scale, Log scale, and Biexponential scale. Examples of the axis scale of the display also include Linear scale, Log scale, and Biexponential scale.

In the present disclosure, the data analysis client terminal 20 may adopt any of these as the axis scale of pie display in each node. For example, in a case where Biexponential scale is adopted as the axis scale of a two-dimensional plot, the data analysis client terminal 20 can adopt Biexponential scale as the axis scale of pie display of one or more nodes corresponding to the two-dimensional plot.

In the present disclosure, in order to change axis settings described above, the data analysis client terminal 20 may be configured to be able to output a two-dimensional plot settings window. Supposed is a case where, as output data, a two-dimensional plot data 710 depicted in FIG. 39 is caused to be displayed on the output apparatus by the data analysis client terminal 20, for example.

In this case, for example, in response to execution of a predetermined operation by a user, the data analysis client terminal 20 causes the output apparatus to display a plot settings window 711. As depicted in FIG. 39, the plot settings window has areas 712 and 713 for adjusting settings of the X axis and the Y axis of the two-dimensional plot data 710. As depicted in FIG. 39, the area 712 for setting the X axis has a list box 714 for selecting the axis scale of the X axis. Whereas the list box displays "Biexponential" in FIG. 39, in addition to "Biexponential," the list box is configured to also allow selection of "Linear" and "Log." The area 713 for setting the Y axis also has a list box 715 for selecting the axis scale of the Y axis similarly. The list box also is configured to allow selection of any of the three axis scales similarly.

When a user selects the axis scale(s) of the X axis and/or the Y axis on the plot settings window, in response to selection of the axis scale(s), the data analysis client terminal 20 can change the axis scale(s) of pie display of one or more nodes corresponding to the two-dimensional plot controlled according to the plot settings window. The change makes the axis scale(s) of the pie display the same as the axis scale(s) selected in the plot settings.

The present disclosure provides the information processing system described above as well as a server system, a data acquisition client terminal, and a data analysis client terminal that are included in the information processing system. Details of these are as explained above.

2. Information Processing Method

The present disclosure also provides an information processing method. The information processing method may include one or more processes in the processes explained in (4) above. For example, the information processing method may include an automatic analysis process step of performing an analysis process on optical intensity data acquired by irradiation of a biosample with light or fluorescence label intensity data calculated from the optical intensity data and generating to-be-output data, an analysis result data storage step of storing the to-be-output data generated on the basis of the optical intensity data or the fluorescence label intensity data, and an interactive analysis process step of analyzing the fluorescence label intensity data on the basis of an analysis command for the to-be-output data output to an output apparatus and outputting analysis result data. The explanation in (4) above holds true for each of these steps.

Note that the present disclosure can also adopt a configuration like the ones below.

[1] A server system including:

an automatic analysis processing section that generates to-be-output data by an analysis process on optical intensity data or fluorescence label intensity data acquired by irradiation of a biosample with light;
an analysis result data storage section that stores the to-be-output data generated on the basis of the optical intensity data or the fluorescence label intensity data; and
an interactive analysis processing section that analyzes the fluorescence label intensity data on the basis of an analysis command for the to-be-output data
output to an output apparatus and outputs analysis result data.

[2] The server system according to [1], in which the automatic analysis processing section calculates fluorescence label intensity data from the optical intensity data.
[3] The server system according to [1] or [2], in which a process by the automatic analysis processing section and a process by the interactive analysis processing section are executed on mutually different computation resources.
[4] The server system according to any one of [1] to [3], in which the server system reserves a computation resource for a process by the automatic analysis processing section and/or a process by the interactive analysis processing section in response to reception of an analysis start command.
[5] The server system according to any one of [1] to [4], further including:
a database on which analysis settings data to be used in a process by the automatic analysis processing section and/or a process by the interactive analysis processing section is stored.
[6] The server system according to any one of [1] to [5], further including:
an optical data storage section that stores the optical intensity data and/or the fluorescence label intensity data.
[7] The server system according to [6], in which

the optical data storage section includes two or more types of storages with different access speeds, and
the server system executes a process of migrating the optical intensity data and/or the fluorescence label intensity data stored on a storage with a higher access speed to a storage with a lower access speed, in a case where a predetermined condition is satisfied.

[8] The server system according to any one of [1] to [7], in which the to-be-output data includes at least one of a two-dimensional plot image, a spectral plot image, a one-dimensional histogram image, a two-dimensional contour plot image, a dimensional compressed image, and a clustering result display view.

[9] An information processing system including:

a data acquisition client terminal that acquires optical intensity data acquired by irradiation of a biosample with light or acquires fluorescence label intensity data by a calculation process on the optical intensity data; and a server system including

an optical data storage section that stores the optical intensity data or the fluorescence label intensity data transmitted from the data acquisition client terminal,
an automatic analysis processing section that generates to-be-output data by an analysis process on the optical intensity data or the fluorescence label intensity data,
an analysis result data storage section that stores the to-be-output data generated on the basis of the optical intensity data or the fluorescence label intensity data, and
an interactive analysis processing section that analyzes the fluorescence label intensity data on the basis of an analysis command for the to-be-output data output to an output apparatus and outputs analysis result data.

[10] The information processing system according to [9], in which, in response to acquisition of the optical intensity data or the fluorescence label intensity data, the data acquisition client terminal transmits the optical intensity data or the fluorescence label intensity data to the server system.

[11] The information processing system according to [9] or [10], in which, in response to acquisition of the optical intensity data or the fluorescence label intensity data, the data acquisition client terminal performs a predetermined process on the optical intensity data or the fluorescence label intensity data, and then transmits the processed optical intensity data or the processed fluorescence label intensity data to the server system.

[12] The information processing system according to any one of [9] to [11], in which

the server system retains in advance analysis settings data to be used in a process by the automatic analysis processing section, and
the automatic analysis processing section calculates the fluorescence label intensity data from the optical intensity data by using the analysis settings data.

[13] The information processing system according to any one of [9] to [12], in which, in response to storage of the optical intensity data on the optical data storage section, the automatic analysis processing section executes a process of calculating fluorescence label intensity data from the optical intensity data.

[14] The information processing system according to any one of [9] to [13], further including:
a data analysis client terminal including the output apparatus.

[15] The information processing system according to [14], in which the data analysis client terminal transmits, to the server system, the analysis command for the to-be-output data output to the output apparatus.

[16] The information processing system according to [14] or [15], in which the data analysis client terminal causes the output apparatus to output a window on which the to-be-output data is displayed, and accepts input of the analysis command on the window.

[17] The information processing system according to any one of [9] to [16], in which multiple data analysis client terminals are able to share any one or more of optical intensity data, fluorescence intensity data, and analysis settings data in the server system.

[18] The information processing system according to any one of [9] to [17], in which multiple data acquisition client terminals are able to share analysis settings data in the server system.

[19] A data acquisition client terminal including:

a data acquiring section that acquires optical intensity data acquired by irradiation of a biosample with light; and
a transmitting section that transmits the optical intensity data to a server system in response to acquisition of the optical intensity data, in which,
in the server system, fluorescence label intensity data is calculated from the optical intensity data.

[20] A data analysis client terminal including:

a communication section that receives, from a server system, to-be-output data created by the server system on the basis of fluorescence label intensity data; and

a processing section that performs a process of causing an output apparatus to output the to-be-output data.

[21] The data analysis client terminal according to [20], in which the data analysis client terminal causes the output apparatus to output a window on which the to-be-output data is displayed, and accepts input of an analysis command for the to-be-output data on the window.

[22] An information processing method including:

an automatic analysis process step of performing an analysis process on optical intensity data acquired by irradiation of a biosample with light or fluorescence label intensity data calculated from the optical intensity data and generating to-be-output data;

an analysis result data storage step of storing the to-be-output data generated on the basis of the optical intensity data or the fluorescence label intensity data; and

an interactive analysis process step of analyzing the fluorescence label intensity data on the basis of an analysis command for the to-be-output data output to an output apparatus and outputting analysis result data.

[Reference Signs List]

1: Information processing system
10: Server system
20: Data analysis client terminal
30: Data acquisition client terminal
40: Biosample analyzing apparatus

**Claims**

1. A server system comprising:

an automatic analysis processing section that generates to-be-output data by an analysis process on optical intensity data or fluorescence label intensity data acquired by irradiation of a biosample with light;

an analysis result data storage section that stores the to-be-output data generated on a basis of the optical intensity data or the fluorescence label intensity data; and

an interactive analysis processing section that analyzes the fluorescence label intensity data on a basis of an analysis command for the to-be-output data output to an output apparatus and outputs analysis result data.

2. The server system according to claim 1, wherein the automatic analysis processing section calculates fluorescence label intensity data from the optical intensity data.

3. The server system according to claim 1, wherein a process by the automatic analysis processing section and a process by the interactive analysis processing section are executed on mutually different computation resources.

4. The server system according to claim 1, wherein the server system reserves a computation resource for a process by the automatic analysis processing section and/or a process by the interactive analysis processing section in response to reception of an analysis start command.

5. The server system according to claim 1, further comprising:
a database on which analysis settings data to be used in a process by the automatic analysis processing section and/or a process by the interactive analysis processing section is stored.

6. The server system according to claim 1, further comprising:
an optical data storage section that stores the optical intensity data and/or the fluorescence label intensity data.

7. The server system according to claim 6, wherein

the optical data storage section includes two or more types of storages with different access speeds, and
the server system executes a process of migrating the optical intensity data and/or the fluorescence label intensity data stored on a storage with a higher access speed to a storage with a lower access speed, in a case where a predetermined condition is satisfied.

8. The server system according to claim 1, wherein the to-be-output data includes at least one of a two-dimensional plot image, a spectral plot image, a one-dimensional histogram image, a two-dimensional contour plot image, a dimensional compressed image, and a clustering result display view.

9. An information processing system comprising:

a data acquisition client terminal that acquires optical intensity data acquired by irradiation of a biosample with light or acquires fluorescence label intensity data by a calculation process on the optical intensity data; and
a server system including

an optical data storage section that stores the optical intensity data or the fluorescence label intensity data transmitted from the data acquisition client terminal,
an automatic analysis processing section that generates to-be-output data by an analysis process on the optical intensity data or the fluorescence label intensity data,
an analysis result data storage section that stores the to-be-output data generated on a basis of the optical intensity data or the fluorescence label intensity data, and
an interactive analysis processing section that analyzes the fluorescence label intensity data on a basis of an analysis command for the to-be-output data output to an output apparatus and outputs analysis result data.

10. The information processing system according to claim 9, wherein, in response to acquisition of the optical intensity data or the fluorescence label intensity data, the data acquisition client terminal transmits the optical intensity data or the fluorescence label intensity data to the server system.

11. The information processing system according to claim 9, wherein, in response to acquisition of the optical intensity data or the fluorescence label intensity data, the data acquisition client terminal performs a predetermined process on the optical intensity data or the fluorescence label intensity data, and then transmits the processed optical intensity data or the processed fluorescence label intensity data to the server system.

12. The information processing system according to claim 9, wherein

the server system retains in advance analysis settings data to be used in a process by the automatic analysis processing section, and
the automatic analysis processing section calculates the fluorescence label intensity data from the optical intensity data by using the analysis settings data.

13. The information processing system according to claim 9, wherein, in response to storage of the optical intensity data on the optical data storage section, the automatic analysis processing section executes a process of calculating fluorescence label intensity data from the optical intensity data.

14. The information processing system according to claim 9, further comprising:
a data analysis client terminal including the output apparatus.

15. The information processing system according to claim 14, wherein the data analysis client terminal transmits, to the server system, the analysis command for the to-be-output data output to the output apparatus.

16. The information processing system according to claim 14, wherein the data analysis client terminal causes the output apparatus to output a window on which the to-be-output data is displayed, and accepts input of the analysis command on the window.

17. The information processing system according to claim 9, wherein multiple data analysis client terminals are able to share one or more of optical intensity data, fluorescence label intensity data, and analysis settings data in the server system.

18. The information processing system according to claim 9, wherein multiple data acquisition client terminals are able to share analysis settings data in the server system.

**19.** A data acquisition client terminal comprising:

a data acquiring section that acquires optical intensity data acquired by irradiation of a biosample with light; and
a transmitting section that transmits the optical intensity data to a server system in response to acquisition of the optical intensity data, wherein,
in the server system, fluorescence label intensity data is calculated from the optical intensity data.

**20.** A data analysis client terminal comprising:

a communication section that receives, from a server system, to-be-output data created by the server system on a basis of fluorescence label intensity data; and
a processing section that performs a process of causing an output apparatus to output the to-be-output data.

**21.** The data analysis client terminal according to claim 20, wherein the data analysis client terminal causes the output apparatus to output a window on which the to-be-output data is displayed, and accepts input of an analysis command for the to-be-output data on the window.

**22.** An information processing method comprising:

an automatic analysis process step of performing an analysis process on optical intensity data acquired by irradiation of a biosample with light or fluorescence label intensity data calculated from the optical intensity data and generating to-be-output data;
an analysis result data storage step of storing the to-be-output data generated on a basis of the optical intensity data or the fluorescence label intensity data; and
an interactive analysis process step of analyzing the fluorescence label intensity data on a basis of an analysis command for the to-be-output data output to an output apparatus and outputting analysis result data.

# FIG.1

# FIG.2

# FIG.3

FIG.4

CD34$^+$ CD38$^-$  HSC
CD45RA$^-$
CD49$^+$
CD90/Thy$^+$

MPP
CD45RA$^-$
CD90/Thy$^-$

CD34$^+$ CD38$^+$

CMP
CD7$^-$
CD10$^-$
CD45RA$^-$
CD90·Thy1$^-$
CD135$^+$

CLP
CD10$^+$
CD45RA$^+$

MEP
CD7$^-$
CD10$^-$
CD45RA$^-$
CD135$^-$
IL3Ra$^-$

GMP
CD10$^-$
CD45RA$^+$
CD123$^+$
CD135$^+$

**Mega karyocyte**
CD41b$^+$
CD42a$^+$
CD42b$^+$
CD61$^-$

**Platelet**
CD41$^+$
CD42a$^+$
CD42b$^+$
CD61$^+$

**Erythrocyte**
CD235a$^+$

**Neutrophil**
CD11b$^-$
CD16$^+$
CD18$^+$
CD32$^+$
CD44$^+$
CD55$^+$

**Eosinophil**
CD45$^+$
CD125$^+$
CD193$^+$
F4/80$^+$
Siglec-8$^+$

**Basophil**
CD19$^-$
CD22$^+$
CD45$^{kw}$
CD123$^+$

**Mast cell**
CD32$^+$
CD33$^+$
CD117$^+$
CD203c$^+$
FceRI$^+$

**Monocyte**∗
CD14$^+$

**Macrophage**
CD11b$^+$
CD68$^+$
CD163$^+$

**Dendritic cell**
CD11$^+$
HLA-DR$^+$

**NK cell**∗
CD3$^-$
CD56$^+$
CD94$^+$
NKp46$^+$

**T cell**
CD3$^+$

**B cell**
CD19$^+$

# FIG.5

SERVER SYSTEM

ANALYZING APPARATUS

DATA ACQUISITION CLIENT TERMINAL

DATA ANALYSIS CLIENT TERMINAL

# FIG.6

CONNECTING SECTION

OPTICAL DATA STORAGE SECTION

ANALYSIS RESULT DATA STORAGE SECTION

DATABASE

AUTOMATIC ANALYSIS PROCESSING SECTION

INTERACTIVE ANALYSIS PROCESSING SECTION

TO-BE-OUTPUT DATA GENERATING SECTION

FIG. 7

FIG.8

# FIG.9

# FIG.10

# FIG.11

# FIG.12

# F I G . 1 3

LIGHT
APPLYING
SECTION
101

SENSING
SECTION
102

INFORMATION
PROCESSING
SECTION
103

ISOLATING
SECTION
104

40

S

P

C

# F I G . 1 4

| DATA ACQUISITION CLIENT TERMINAL | SERVER SYSTEM | DATA ANALYSIS CLIENT TERMINAL |

**S101**
ACQUIRE OPTICAL INTENSITY DATA

**S102**
TRANSMIT OPTICAL INTENSITY DATA

**S103**
RECEIVE OPTICAL INTENSITY DATA

**S104**
AUTOMATIC ANALYSIS PROCESS

**S105**
TRANSMIT ANALYSIS COMPLETION NOTIFICATION

**S106**
RECEIVE ANALYSIS COMPLETION NOTIFICATION

**S108**
RECEIVE REQUEST FOR TO-BE-OUTPUT DATA

**S107**
TRANSMIT REQUEST FOR TO-BE-OUTPUT DATA

**S109**
TRANSMIT TO-BE-OUTPUT DATA

**S110**
RECEIVE TO-BE-OUTPUT DATA

**S111**
OUTPUT TO-BE-OUTPUT DATA

EP 4 227 665 A1

# FIG.15

START — S151

RESERVE COMPUTATION RESOURCES — S152

DOWNLOAD OPTICAL INTENSITY DATA — S153

DOWNLOAD DATA NECESSARY FOR ANALYSIS — S154

EXECUTE ANALYSIS — S155

GENERATE TO-BE-OUTPUT DATA — S156

END — S157

# FIG.16

```
┌─────────────────────┐                    ┌──────────────────────────────┐
│    SERVER SYSTEM    │                    │  DATA ANALYSIS CLIENT TERMINAL │
└─────────────────────┘                    └──────────────────────────────┘
```

S202                                          S201

RECEIVE REQUEST FOR          ◄──────────    TRANSMIT REQUEST FOR
TO-BE-OUTPUT DATA                            TO-BE-OUTPUT DATA

S203

ACQUIRE TO-BE-OUTPUT DATA

S204                                          S205

TRANSMIT TO-BE-OUTPUT DATA   ──────────►    RECEIVE TO-BE-OUTPUT DATA

                                              S206

                                             OUTPUT TO-BE-OUTPUT DATA

# FIG.17

| SERVER SYSTEM | | DATA ANALYSIS CLIENT TERMINAL |
|---|---|---|

S302
RECEIVE PROCESSING START REQUEST ◄—————

S301
TRANSMIT PROCESSING START REQUEST

S303
RESERVE RESOURCES

S304
ACQUIRE ANALYSIS-SUBJECT DATA

S305
ACCEPT ANALYSIS COMMAND INPUT

S307
RECEIVE ANALYSIS COMMAND ◄—————

S306
TRANSMIT ANALYSIS COMMAND

S308
EXECUTE ANALYSIS

S309
GENERATE AND TRANSMIT TO-BE-OUTPUT DATA —————►

S310
RECEIVE TO-BE-OUTPUT DATA

S311
OUTPUT TO-BE-OUTPUT DATA

REPEAT S305 TO S311

# FIG.18

Lambda — S303 → ECS

S302

S301

S303

EC2

Container ← S304 — S3

FIG.19

S311

S306      S307

S310      S309

S305

EC2

S308

Container

S308

S3

EP 4 227 665 A1

# FIG.20

EP 4 227 665 A1

# FIG. 21

FIG.22

Sample Group

☑ Sample Group 1
☐ Sample Group 2
☐ Sample Group 3
·
·
·

Sample

|   | 01 | 02 | 03 | 04 | 05 | 06 | 07 | 08 | 09 | 10 | 11 | 12 |
|---|----|----|----|----|----|----|----|----|----|----|----|----|
| A | ✓ | ✓ | ✓ | ✓ | ✓ | ✓ | ✓ | ✓ | ✓ | ✓ | ✓ | ✓ |
| B | ✓ | ✓ | ✓ | ✓ | ✓ | ✓ |   |   |   |   |   |   |
| C |   |   |   |   |   |   |   |   |   |   |   |   |
| D |   |   |   |   |   |   |   |   |   |   |   |   |
| E |   |   |   |   |   |   |   |   |   |   |   |   |
| F |   |   |   |   |   |   |   |   |   |   |   |   |
| G |   |   |   |   |   |   |   |   |   |   |   |   |
| H |   |   |   |   |   |   |   |   |   |   |   |   |

| Marker | FC |
|--------|-----|
| CD16 | BV421 |
| CD3 | FITC |
| CD45 | BV570 |
| · | · |
| · | · |

Unmixing

BV421 · · · ·
FITC · · · ·
BV570 · · · ·
·
·

Setting

Laser:
Detector:
·
·

# FIG.23

EP 4 227 665 A1

**Gates and Statistics**

| Name | Event | Parent% | Total% |
|---|---|---|---|
| CD3+ | 82,142 | 36.45% | 20.41% |
| Cytotoxic T cells CD8+ | 26,267 | 31.98% | 6.53% |
| CD45RA+CCR7- | 13,168 | 50.13% | 3.27% |
| CD45RA+CCR7+ | 1,188 | 4.52% | 0.30% |
| CD45RA-CCR7+ | 2,392 | 9.11% | 0.59% |
| Helper T cells CD4+ | 40,321 | 49.09% | 10.02% |
| Regulatory T cells | 1,280 | 3.17% | 0.32% |
| Activated T cells | 482 | 37.66% | 0.12% |
| CD4+ CD45RA+ CCR7- | 210 | 0.52% | 0.05% |
| CD4+ CD45RA+ CCR7+ | 3,631 | 9.01% | 0.90% |
| CD4+ CD45RA- CCR7+ | 21,896 | 54.30% | 5.44% |
| AAAL | 1,662 | 4.12% | 0.41% |
| NKT cells | 13,466 | 16.39% | 3.35% |

(Monocytes gate)

(Lymphocytes gate)
B cells
T cells

(Lymphocytes gate)
NK cells

(CD19+ gate)
Memory B cells

(CD3+ gate)
Th cells
Th cells

(CD3+CD4+ gate)
Naive T cells
Memory T cells

FIG.24

# FIG.25

# FIG.26

F I G . 2 7

EP 4 227 665 A1

514

510

# FIG.28

523

Star Chart Legend - W

PD-1_BV421_A
IgD_BV480_A
HLA-DR_BV570_A
CXCR5_BV750_A
CD86_BB515_A

CCR6_BV711_A
CD14_BUV563_A
CD27_APC_A
CD57_FITC_A
CD8_QD800_A

Fluorochromes:
CCR6_BV711_A
CCR7_BV785_A
CD11b_PerCP-Cy5.5_A
CD11c_BUV661_A
CD123_AF700_A
CD127_PE-Cy5.5_A
CD14_BUV563_A
CD16_BUV496_A
CD161_eFluor450_A
CD19_AF532_A
CD20_PacOrange_A
CD24_PE-Dazzle594_A
CD25_PE-Cy7_A
CD27_APC_A
CD28_BV605_A
CD3_BV510_A
CD33_AF647_A
CD335_PE_A
CD38_APC-eFluor780_A
CD4_[CF 568]_A
CD45_PerCP_A
CD45RA_BUV395_A
CD45RO_BUV805_A
CD56_BUV737_A
CD57_FITC_A
CD8_QD800_A
CD86_BB515_A
CD95_PE-Cy5_A
CXCR3_BV650_A
CXCR5_BV750_A
HLA-DR_BV570_A
IgD_BV480_A
PD-1_BV421_A
TCRgd_PerCP-eFluor710_A

Close

521

522

520

F I G . 2 9

# FIG.30

530

F I G . 3 1

FIG.32

600

601

FIG.33

EP 4 227 665 A1

# FIG.34

EP 4 227 665 A1

F I G . 3 5

EP 4 227 665 A1

# FIG.36

EP 4 227 665 A1

FIG.37

FIG.38

700

702

701

F I G . 3 9

Plot Properties

Title:

Source: ■ W

Plot Type: Dot Plot

X Axis
Parameter: CD3_BV510  Area  Biexponential
Axis Type:  ▶ Options

Minimum: -23852
Maximum: 1000000
Label: CD3_BV510_A

714

Y Axis
Parameter: PD-1_BV421  Area  Biexponential
Axis Type:  ▶ Options

Minimum: -154
Maximum: 1000000
Label: PD-1_BV421_A

715

Close

711

712

713

710

WLSM

■ W

PD-1_BV421_A

CD3_BV510_A

# EP 4 227 665 A1

## INTERNATIONAL SEARCH REPORT

| | International application No. |
|---|---|
| | **PCT/JP2021/034791** |

### A. CLASSIFICATION OF SUBJECT MATTER

*G01N 15/14*(2006.01)i; *G01N 21/64*(2006.01)i
FI:    G01N15/14 B; G01N15/14 C; G01N21/64 Z; G01N21/64 F

According to International Patent Classification (IPC) or to both national classification and IPC

### B. FIELDS SEARCHED

Minimum documentation searched (classification system followed by classification symbols)

G01N15/14; G01N21/64

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| Y | JP 2015-171333 A (FUJITSU LTD.) 01 October 2015 (2015-10-01) paragraphs [0001]-[0067], fig. 1, 2 | 1-22 |
| Y | WO 2014/141878 A1 (SONY CORP.) 18 September 2014 (2014-09-18) paragraphs [0001]-[0054], fig. 1-25 | 1-22 |
| Y | JP 2006-309288 A (SONY CORP.) 09 November 2006 (2006-11-09) paragraph [0002] | 7, 16, 21 |
| Y | JP 2020-56593 A (SYSMEX CORP.) 09 April 2020 (2020-04-09) claims, fig. 5-48 | 16,21 |
| A | JP 2005-265686 A (KURITA WATER INDUSTRIES LTD.) 29 September 2005 (2005-09-29) entire text, all drawings | 1-22 |
| A | JP 2003-232796 A (MATSUSHITA ELECTRIC INDUSTRIAL CO., LTD.) 22 August 2003 (2003-08-22) entire text, all drawings | 1-22 |

✓ Further documents are listed in the continuation of Box C.      ✓ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **26 November 2021** | **07 December 2021** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**

International application No.

**PCT/JP2021/034791**

| C. | DOCUMENTS CONSIDERED TO BE RELEVANT | |
|---|---|---|
| Category* | Citation of document, with indication, where appropriate, of the relevant passages | Relevant to claim No. |
| A | US 2006/0015291 A1 (LELAND STANFORD JUNIOR UNIVERSITY) 19 January 2006 (2006-01-19) entire text, all drawings | 1-22 |

Form PCT/ISA/210 (second sheet) (January 2015)

INTERNATIONAL SEARCH REPORT
Information on patent family members

International application No.

**PCT/JP2021/034791**

| Patent document cited in search report | | | Publication date (day/month/year) | Patent family member(s) | Publication date (day/month/year) |
|---|---|---|---|---|---|
| JP | 2015-171333 | A | 01 October 2015 | (Family: none) | |
| WO | 2014/141878 | A1 | 18 September 2014 | US 2016/0011095 A1 paragraphs [0001]-[0115], fig. 1-25 EP 2975384 A1 CN 105074435 A | |
| JP | 2006-309288 | A | 09 November 2006 | (Family: none) | |
| JP | 2020-56593 | A | 09 April 2020 | US 2020/0103333 A1 claims, fig. 5-48 EP 3629002 A1 CN 110967512 A | |
| JP | 2005-265686 | A | 29 September 2005 | (Family: none) | |
| JP | 2003-232796 | A | 22 August 2003 | (Family: none) | |
| US | 2006/0015291 | A1 | 19 January 2006 | (Family: none) | |

Form PCT/ISA/210 (patent family annex) (January 2015)

**REFERENCES CITED IN THE DESCRIPTION**

**Patent documents cited in the description**

- JP 2018527674 T
- JP 2020051838 A

- JP 5985140 B